# EUROPEAN PATENT SPECIFICATION

(11) **EP 3 315 199 B1**
(45) Date of publication and mention of the grant of the patent: **29.07.2020**
(21) Application number: 17198861.1
(22) Date of filing: 27.10.2017
(51) Int. Cl.: B01L 3/00

(54) **MEASURING CARTRIDGE AND LIQUID TRANSPORT METHOD**
MESSKARTUSCHE UND FLÜSSIGKEITSTRANSPORTVERFAHREN
CARTOUCHE DE MESURE ET PROCÉDÉ DE TRANSPORT DE LIQUIDES

(30) Priority: 31.10.2016 JP 2016212860
(43) Date of publication of application: 02.05.2018
(73) Proprietor: SYSMEX CORPORATION, Kobe-shi Hyogo 651-0073 (JP)
(72) Inventor: Nose, Tomoyuki, Hyogo, 651-0073 (JP); Horii, Kazuyoshi, Hyogo, 651-0073 (JP); Tomoda, Sayuri, Hyogo, 651-0073 (JP)
(74) Representative: Hoffmann Eitle

(56) References cited:
- US-A1- 2009 298 092
- US-A1- 2012 295 781
- US-B2- 7 854 893

## Description

### FIELD OF THE INVENTION

The invention relates to a measuring cartridge and liquid transport method for separating a liquid component from a sample by centrifugation and using the separated component for measurement.

### BACKGROUND

U.S. Patent Application Publication No. 2010/0240142 discloses a configuration in which a sample is analyzed using a disc-shaped biological analysis device.

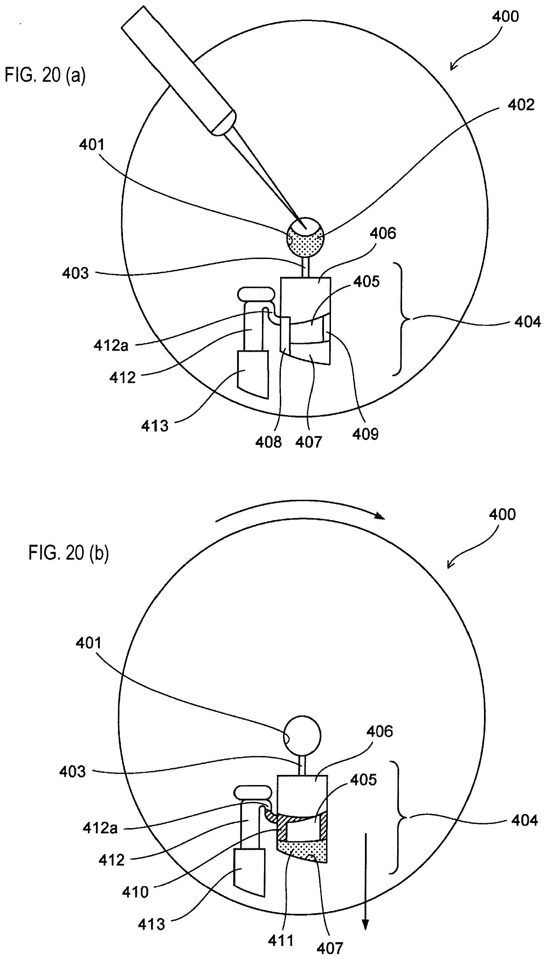

Specifically, as shown in FIG. 20 (a), the blood 402 injected into the blood reservoir 401 is subjected to the centrifugal force generated by the rotation of the biological analysis device 400 and transferred into a blood separation part 404. The interior of the blood separation part 404 is divided by a blood separation wall 405 into a plasma reservoir 406 and a blood cell reservoir 407. A plasma collection capillary 408 and a ventilation flow path 409 are formed in the blood separation wall 405 so as to connect the plasma reservoir 406 and the blood cell reservoir 407. As shown in FIG. 20 (b), the blood 402 transferred to the blood separation part 404 is further separated into a plasma component 410 and a blood cell component 411 by centrifugal force. Thereafter, by decelerating or stopping the rotation of the biological analysis device 400, the separated plasma component 410 is transported to the plasma measuring unit 412 through the siphon flow path 412a via capillary action. Subsequently, the plasma component 410 is transferred to the reagent reaction unit 413 by centrifugal force, and is used for analysis. US 7,854,893 B2, US 2012/0295781 A1, and US 2009/0298092 A1 are prior art documents relating to sample analysers.

### SUMMARY OF THE INVENTION

In the configuration disclosed in U.S. Patent Application Publication No. 20100240142, the blood cell component 411 tends to remain on the wall surface on the rotating shaft side of the blood separation wall 405 during centrifugal separation. Due to this factor, if the blood cell component 411 remains in the plasma reservoir 406 after centrifugation, the residual blood cell component 411 may be mixed into the plasma measuring unit 412 via the siphon flow path 412a, and the analysis accuracy of the plasma component 410 may be lowered.

In view of such problems, the present invention provides a transport method and measuring cartridge capable of improving measurement accuracy of plasma components by suppressing mixing of blood cell components when transferring plasma components separated by centrifugation.

The invention is defined by the independent claims.

A first aspect of the invention relates to a measuring cartridge (10, 200, 300) installed in a measuring device (100) capable of rotation on a rotating shaft (20, 103).

The measuring cartridge (10, 200, 320) of this aspect includes a sample input port (61, 201) into which a blood sample (70, 280) is introduced, a first storage part (31, 211), a second storage part (32, 212) disposed in a direction away from the rotating shaft (20, 103) relative to the first storage part (31, 211) and having a larger width in the rotation direction around the rotating shaft (20, 103) than that in the first storage part (31, 211), a separation chamber (30, 210) that separates a blood sample (70, 280) into a blood cell component (71, 281) and blood plasma component (71, 281) by utilizing centrifugal force through rotation around the rotating shaft (20, 103), receiving chamber (40, 241) for receiving the plasma component (72, 282), flow paths (50, 51, 52, 63, 203, 220, 221, 222) connected to one of the inner wall (31a, 31b, 32a, 32b, 81a, 81b, 211a, 212a, 271a) of at least the first storage part (31, 211) or the second storage part (32, 212), wherein the flow path (50, 63, 203, 220) includes a first flow path (63, 203) for moving the blood sample (70, 280) from the sample input port (61, 201) to the separation chamber (30, 210), and a second flow path (50, 220) for moving the separated plasma component (72, 282) in the separation chamber (30, 210) by capillary action.

In the measuring cartridge of this aspect, the blood sample is separated into a blood cell component and a plasma component by centrifugal separation in the separation chamber. At this time, the interface between the plasma component and the air layer appearing in the first storage part after the centrifugal separation is greatly distanced from the rotating shaft side relative to the second storage part, since the width of the first storage part is shorter than that of the second storage part in the rotation direction. Therefore, it is possible to widen the distance between the interface and the liquid layer of the blood cell component after centrifugation, and the second flow path can be connected to the inner wall of the first storage part or the second storage part that is distant from the liquid layer of the blood cell component. This makes it possible to prevent the blood cell component after centrifugation from being included in the flow of the plasma component from the separation chamber to the second flow path, and prevents the blood cell component from contaminating the plasma component in the second flow path. According to the measuring cartridge of this aspect, it is therefore possible to suppress the mixing of the blood cell component with the plasma component moving in the second flow path from the separation chamber to the receiving chamber, thus enhancing the measurement accuracy for the plasma component.

In the measuring cartridge (10, 200, 320) according to this aspect, the second flow path (50, 220) is configured to be connected to the first inner wall (32 a, 32 b, 212 a) located at the end on the rotating shaft (20, 103) side of the second storage part (32, 212). In this way, the second flow path can extend from the connection position with the first inner wall along the radial direction of the circle around the rotating shaft. This makes it possible to smoothly move the blood cell component collected in the second flow path before centrifugation to the first storage part by the centrifugal force during centrifugal separation. Therefore, it is possible to more reliably prevent residual blood cell components remaining in the second flow path after centrifugation. The second flow path connected to the second storage part also can be kept away from the liquid layer of the blood cell component. Therefore, mixing of blood cell components into plasma components moving in the second flow path from the separation chamber to the receiving chamber can be effectively suppressed.

In this case, the second inner wall (31a, 31b, 211a) of the first storage part (31, 211), which is positioned on the opposite side of the rotating shaft from end part of the first storage part (31, 211) located on the side of the rotating shaft (20, 103), is configured to be inclined so as to gradually become parallel to the first inner wall (32a, 32b, 212a) from the end edge of the second inner wall (31a, 31b, 211a) to the first inner wall (32a, 32b, 212a) by a curved inner wall (211b) leading to the first inner wall (32a, 32b, 212a), and be connected to the first inner wall (32a, 32b, 212a) by a curved inner wall (211b) leading to the first inner wall (32a, 32b, 212a). In this way, when the plasma component moves from the second storage part to the second flow path, turbulence of the plasma component is suppressed from occurring in the vicinity of the end part of the first inner wall on the side of the first storage part because the flow of the plasma component smoothly changes along the curved inner wall. Therefore, it is possible to suppress mixing of blood cell components into the second flow path due to such a turbulent flow.

In the measuring cartridge (10, 200, 320) according to this aspect, the second flow path (50, 220) is configured so as to connect to the inner wall (31a, 31b, 211a) of the first storage part (31, 211) located on the opposite side of the rotating shaft (20, 103) from the end part of the first storage part (31, 211) located on the rotation shaft (20, 103) side.

In the measuring cartridge (10, 200, 300) according to this aspect, the second flow path (50, 220) may be configured so as to extend from the separation chamber (30, 210) in a direction toward the rotating shaft (20, 103). In this case, since the second flow path extends in a direction away from the separation chamber toward the rotating shaft, the blood cell component entering the second flow path from the separation chamber before centrifugation is separated by the centrifugal force during centrifugal separation and transported from the second flow path to the separation chamber. Therefore, it is possible to more reliably prevent residual blood cell components remaining in the second flow path after centrifugation. Note that the second flow path may be extended in a direction toward the rotating shaft, but need not necessarily extend exactly toward the rotating shaft. The second flow path also need not necessarily extend in a direction toward the rotating shaft over the entire length, but may extend in a direction toward the rotating shaft at least within a range where the blood sample can enter before centrifugal separation.

In the measuring cartridge (10, 200, 320) according to this aspect, the second storage part (32, 212) is configured with a first area (81, 271), and a second area (82, 272) that is smaller than the first area (81, 272) and disposed on the rotating shaft (20, 103) side relative to the first area (81, 271). In this way, the blood cell component can be efficiently accommodated in the wide first area during centrifugal separation. The interface between the air layer after centrifugation and the plasma component also is located closer to the rotating shaft side since the width of the second area is smaller than that of the first area. In this way, it is possible to more reliably suppress the blood cell component from being involved in the flow of the plasma component into the flow path.

In this case, the first storage part (31, 211) is disposed at a position biased in one direction of the rotation direction relative to the second area (82, 272), and the second flow path (50, 200) is connected to the first storage part (31, 211) at the position of the second are (82, 272) on the opposite side of this direction. In this way, while the plasma component flows into the second flow path, the interface between the air layer and the plasma component advances in a direction away from the rotating shaft while in a state in which the end on the second flow path side is closer to the rotation axis than the end on the opposite side of the second flow path. In this way, the plasma component can be prevented from moving away from the inlet of the second flow path, and the plasma component can be stably and continuously supplied into the second flow path while the plasma component flows into the second flow path.

In this case, the first area (81, 271) also is configured to have a third inner wall (81a, 81b, 271a) positioned at the end on the rotating shaft (20, 103) side of the first area (81, 271), and connected to the second area (82, 272).

In this case, the third inner wall (81 a, 81 b, 271 a) may be configured longer than the first inner wall (32 a, 32 b, 212 a) in the rotation direction. In this configuration, a part of the first area projecting in the rotational direction relative to the second area is lengthened. Hereinafter, the part of the first area protruding in the rotation direction with respect to the second area is referred to as a "protruding part". The blood cell component accumulated in the protruding part of the first area by centrifugation is not easily influenced by the flow of the plasma component and tends to remain in the protruding part even after centrifugation. Therefore, the blood cell component accumulated in the protruding part by centrifugation scarcely enters the flow of the plasma component into the second flow path. Accordingly, it is possible to increase the amount of the blood cell component collected in the protruding part by increasing the length of the protruding part of the first area by lengthening the third inner wall. Hence, it is possible to more effectively prevent centrifuged blood cell components from flowing into the second flow path.

In this case, the measuring cartridge (10, 200, 320) according to this aspect is configured to have a waste chamber (234) for discarding the plasma component (72, 282), and another flow path (233) connecting the first area (81, 271) and the waste chamber (234) for transporting the plasma component (72, 282) remaining in the separation chamber (30, 210) after the plasma component (72, 282) has been moved from the separation chamber (30, 210) to the receiving chamber (40, 241) from the first area (81, 271) toward the waste chamber (234) via the siphon principle. In this way, it is possible to prevent the plasma component remaining in the separation chamber from reentering into the second flow path to the receiving chamber after moving the plasma component from the separation chamber to the receiving chamber. It is therefore possible to stabilize the amount of the plasma component transferred to the receiving chamber, and it is possible to improve the measurement accuracy of the plasma component.

In this case, the other flow path (233) may be configured to be connected to the third inner wall (81a, 81b, 271a). In this way, it is possible to prevent the blood cell component from mixing into other flow paths at the time of discarding the plasma component and blocking other flow paths.

In the measuring cartridge (10, 200, 320) according to this aspect, the first storage part (31, 211) is disposed at a position biased in a first direction (T2 direction) along the rotation direction relative to the second area (82, 272), and the second area (82, 272) is disposed at a position biased in a second direction (T1 direction) opposite to the first direction relative to the first area (81, 271).

In the measuring cartridge (10, 200, 320) according to this aspect, the second flow path (50, 200) includes a third flow path (51, 221) extending from the separation chamber (30, 210) in a direction toward the rotating shaft (20, 103) and connecting the separation chamber (30, 210) and the receiving chamber (40, 241), and a fourth flow path extending from the end part on the side opposite the separation chamber (30, 210) of the third flow path (51, 221) in a direction away from the rotating shaft (20, 103).

In this case, an air introduction path (65, 207) capable of introducing air into the second flow path (50, 200) may be connected at the connection position (50a, 220a) between the third flow path and the fourth flow path. In this way, when the measuring cartridge is rotated in a state where the second flow path is filled with the plasma component, air enters the flow path from the air introduction path, and the plasma component that fills the second flow path is separated to the connection position. As a result, the plasma component in the third flow path is returned to the separation chamber by centrifugal force, and the plasma component in the fourth flow path is moved to the receiving chamber by centrifugal force. The quantitativeness of the plasma component moving to the receiving chamber is improved because the plasma component moving to the receiving chamber becomes the plasma component that fills in the fourth flow path.

A valve (208c) for stopping the movement of the plasma component (72, 282) through capillary action also may be provided on the receiving chamber (40, 241) side of the fourth flow path (52, 222). In this way, the plasma component can be stored in the range of the fourth flow path from the connection position of the third flow path and the fourth flow path to the valve, and the amount of the plasma component defined in this range is transferred to the receiving chamber. Hence, the quantitativeness of the plasma component moving to the receiving chamber is further improved.

In the measuring cartridge (10, 200, 320) according to this aspect, the second storage part (32, 212) is configured to have an inclined part (274a) having a thickness that increases the space in the second storage part (32, 212) as the distance increases therefrom. Increasing the thickness of the area on the radially outer side of the first inclined part to increase the capacity of this area makes it easier for the blood cell component to be retained in the outer area due to the boycott effect, thereby enhancing the centrifugation efficiency. The blood cell component also can be efficiently distributed in the area radially outward of the first inclined part since the plasma component can be smoothly moved from the radially outer side to the radially inner side of the first storage part along the first inclined part.

In the measuring cartridge (10, 200, 320) according to this aspect, the second storage part (32, 212) is configured to have a second inclined part (274b) that decreases the thickness of the space in the second storage part (32, 212) as it goes away from the rotating shaft (20, 103). By reducing the thickness of the area radially outward of the second inclined part and narrowing the thickness of this area, it is difficult for the blood cell component that has moved outward in the radial direction from the second inclined part through centrifugal force to return the radially inner side. Hence, it is possible to more effectively prevent centrifuged blood cell components from flowing into the second flow path. The blood cell component also can be smoothly moved outward in the radial direction of the second inclined portion along the second inclined portion during centrifugation.

In the measuring cartridge (10, 200, 320) according to this aspect, a first inclined part (274a) for increasing the thickness of the space in the second storage part (32, 212) as the distance increases away from the rotating shaft (20, 103) also may be provided in the first area (81, 271).

In the measuring cartridge (10, 200, 320) according to this aspect, a second inclined part (274b) for decreasing the thickness of the space in the second storage portion (32, 212) as the distance increases away from the rotating shaft (20, 103) also may be provided in the second area (82, 272).

In the measuring cartridge (10, 200, 320) according to this aspect, the second storage part (32, 212) is configured to have a first inclined part (274a) that increases the thickness of the space in the second storage part (32, 212) as the distance increases from the rotating shaft (20, 103), and a second inclined part (274b) provided on the side closer to the rotating shaft (20, 103) relative to the first inclined part (274a), that reduces the thickness of the space in the second storage part (32, 212) as the distance increases from the rotating shaft (20, 103). In addition to the effects of the first inclined part and the second inclined part described above, since the convex part is disposed between the area where the blood cell component is stored and the area where the plasma component is stored, the blood cell component can be more effectively prevented from contaminating the plasma component.

In the measuring cartridge (10, 200, 320) according to this aspect, the first inclined portion (274a) for increasing the thickness of the space in the second storage part (32, 212) as the distance increases away from the rotating shaft (20, 103) is provided in the first area (81, 271) and the second inclined part (274b) for reducing the thickness of the space in the second storage part (32, 212) as the distance increases away from the rotating shaft (20, 103) is provided in the second area (82, 272).

In this case, a first inclined part (274a) and second inclined part (274b) are provided so that the thickness (HI) of the space in the second storage part (32, 212) in the first area (81, 271) is larger than the width (H2) of the space in the second storage part (32, 212) in the second area (82, 272). In this way, the blood cell component can be more efficiently retained in the first area.

The first inclined part (274a) and the second inclined part (274b) also may be connected by the flat part (274c). In this way, the blood cell component remaining on the outer side in the radial direction of the first inclined part through centrifugal separation passes through the flat part to prevent mixing with the plasma component since it becomes difficult for the blood cell component to move in the range where the flat part (274 c) is provided. Hence, the blood cell component is more effectively prevented from mixing with the plasma component taken into the second flow path.

A second aspect of the present invention relates to a measuring cartridge (10, 200, 320) mounted on a measuring device (100) rotatable around a rotating shaft (20, 103). The measuring cartridge (10, 200, 320) according to this aspect includes a separation chamber (30, 210) for separating the blood cell component (71, 281) and the plasma component (72, 282) contained in a blood sample (70, 280) by using the centrifugal force generated by rotating the measuring cartridge, a receiving chamber (40, 241) for containing the plasma component (72, 282), a first flow path (51, 221) extending from the separation chamber (30, 210) in a direction toward the rotating shaft (20, 103), a second flow path (52, 222) connected to the receiving chamber (40, 241) and extending from an end part of the first flow path (51, 221) on a side opposite to the separation chamber (30, 210) in a direction away from the rotating shaft (20, 103), and an air introduction path (65, 207) capable of introducing air into the second flow path (52, 222) from the connection position (50a, 220a) between the first flow path (51, 221) and the second flow path (52, 222).

According to the measuring cartridge of this aspect, when the measuring cartridge is rotated with the first flow path and the second flow path filled with plasma component, air enters the second flow path from the air introduction path, and the plasma component filling the first flow path and the second flow path is divided at the connection position. As a result, the plasma component in the first flow path is returned to the separation chamber by centrifugal force, and the plasma component in the second flow path is moved to the receiving chamber by centrifugal force. The quantitativeness of the plasma component moving to the receiving chamber is improved because the plasma component moving to the receiving chamber becomes the plasma component that filled the second flow path.

In the measuring cartridge (10, 200, 320) according to this aspect, the second flow path (52, 222) is configured so that after the plasma component (72, 282) separated in the separation chamber (30, 210) has filled the second flow path, the plasma component (72, 282) quantified by introducing air into the second flow path (52, 222) from the connection position (50a, 220a) through centrifugal force is stored in the receiving chamber (40, 241).

In the measuring cartridge (10, 200, 320) according to this aspect, a valve (208c) for stopping the movement of the plasma component (72, 282) due to the capillary action is provided on the receiving chamber (40, 241) side of the second flow path (52, 222).

The measuring cartridge (10, 200, 320) according to this aspect includes a waste chamber (234) for discarding the plasma component (72, 282), and another flow path (233) connecting the separation chamber (30, 210) and the waste chamber (234) for transporting the plasma component (72, 282) remaining in the separation chamber (30, 210) after the plasma component (72, 282) has been moved from the separation chamber (30, 210) to the receiving chamber (40, 241) toward the waste chamber (234) via the siphon principle.

A third aspect of the present invention relates to a liquid transport method having the features of claim 13.

According to the liquid transport method of this aspect, the effect obtained is the same as in the first aspect.

In the liquid transport method according to this aspect, the second flow path (50, 51, 52, 220, 221, 222) is connected to the inner wall (32a, 32b, 212a) located at the end on the rotating shaft (20, 103) side of the second storage part (32a, 32b, 212a).

A fourth aspect relates to a liquid transport method. The liquid transfer method according to this aspect includes, separating (S102) a blood sample (70, 280) into a blood cell component (71, 281) and a plasma component (72, 282) using a centrifugal force due to rotation around a rotating shaft (20, 103) in a separation chamber (30, 210) included in a measuring cartridge (10, 200, 320) mounted on a measuring device so as to be rotatable about a rotating shaft (20, 103), filling (S103), by capillary action, a flow path (50, 200) connecting the separation chamber (30, 210) and receiving chamber (40, 241) with the plasma component (72, 282) separated in the separation chamber(30, 210), and transporting (S104) the quantified plasma component (72, 282) to the receiving chamber (40, 241) by introducing air into the flow path (50, 200) by centrifugal force.

According to the liquid transport method of this aspect, the effect obtained is the same as in the second aspect.

In the liquid transport method according to this aspect, the movement of the plasma component (72, 282) through capillary action is stopped (S103) on the storage chamber (40, 241) side of the flow path (50, 200).

In the liquid transport method according to this aspect, the plasma component (72, 282) remaining in the separation chamber (30, 210) after the plasma component (72, 282) is moved from the separation chamber (30, 210) to the receiving chamber (40, 241) 72, 282) is moved from the separation chamber (30, 210) to the waste chamber (234) according to the siphon principle (S104).

According to the present invention, it is possible to prevent the contamination of the blood cell component when transferring the plasma component separated by centrifugation, and to improve the measurement accuracy of the plasma component.

### BRIEF DESCRIPTION OF THE DRAWINGS

FIG. 1 is a schematic diagram showing a configuration of a measuring cartridge according to a first embodiment;
FIG. 2 (a) and 2 (b) are diagrams for describing a procedure for separating a solid component and a liquid component contained in a sample and accommodating a liquid component in a receiving chamber according to the first embodiment.
FIG. 3 (a) and 3 (b) are diagrams for describing a procedure for separating a solid component and a liquid component contained in a sample and accommodating a liquid component in a receiving chamber according to the first embodiment;
FIG. 4 (a) and 4 (b) are schematic diagrams showing a configuration of a measuring cartridge according to the first embodiment;
FIG. 5 (a) is a schematic diagram showing a configuration of a measuring cartridge according to a second embodiment;
FIG. 5 (b) and 5 (c) are schematic diagrams showing a configuration of a measuring cartridge according to a modification of the second embodiment;
FIG. 6 (a) is a schematic diagram showing a configuration of a measuring cartridge according to a third embodiment;
FIG. 6 (b) and 6 (c) are schematic diagrams showing a configuration of a measuring cartridge according to a modification of the third embodiment;
FIG. 7 (a) is a schematic diagram showing a configuration of a measuring cartridge according to a specific structural example of the second embodiment;
FIG. 7 (b) is a schematic diagram showing a configuration of a measuring cartridge according to a specific structural example of the second embodiment;
FIG. 8 is an enlarged view schematically showing a configuration of a part of a measuring cartridge according to a specific configuration example of the second embodiment;
FIG. 9 (a) is a schematic diagram showing C1-C2 cross section according to the specific structural example of the second embodiment; FIG. 9 (b) and (c) are schematic views showing cross section C1-C2 according to a modification of the specific configuration example of the second embodiment;
FIG. 10 (a) is an enlarged view schematically showing a configuration of a valve according to a specific configuration example of the second embodiment;
FIG. 10 (b) to 10 (d) describe how the liquid component is suppressed from entering the receiving chamber through capillary action by the valve according to the specific configuration example of the second embodiment;
FIG. 11 is a diagram showing a configuration a main body part according to a specific configuration example of the second embodiment is viewed from diagonally above, and a view showing a configuration when the lid is viewed diagonally from below;
FIG. 12 is a schematic view of a cross section of a measuring device viewed from the side when sectioned at a plane parallel to a YZ plane passing through a rotating shaft according to a specific configuration example of the second embodiment;
FIG. 13 is a block diagram showing a configuration of a measuring device according to a specific configuration example of the second embodiment;
FIG. 14 is a flowchart showing the operation of a measuring device according to a specific configuration example of the second embodiment;
FIG. 15 is a flowchart showing in detail a process of separating a sample according to a specific configuration example of the second embodiment, and transferring a liquid component to a receiving chamber;
FIG. 16 (a) is a schematic diagram showing a state in which a sample is contained in a sample storage part according to a specific configuration example of the second embodiment;
FIG. 16 (b) is a schematic diagram showing a state in which a sample in a sample storage part has been transferred to a separation chamber according to a specific configuration example of the second embodiment;
FIG. 17 (a) is a schematic diagram showing a state in which a sample in a separation chamber is separated into a solid component and a liquid component by a centrifugal force according to a specific configuration example of the second embodiment;
FIG. 17 (b) is a schematic diagram showing a state in which the liquid component in the separation chamber has been transferred to the flow path according to the specific configuration example of the second embodiment;
FIG. 18 (a) is a schematic diagram showing a state in which a liquid component in a second flow path is being transferred to a receiving chamber according to a specific configuration example of the second embodiment;
FIG. 18 (b) is a schematic diagram showing a state in which the transfer of the liquid component is completed according to the specific configuration example of the second embodiment;
FIG. 19 is a schematic diagram showing a configuration when the supporting member and the measuring cartridge are viewed from above according to a fourth embodiment; and
FIG. 20 is a schematic diagram describing a configuration according to a related art.

### DESCRIPTION OF THE EMBODIMENTS

### First Embodiment

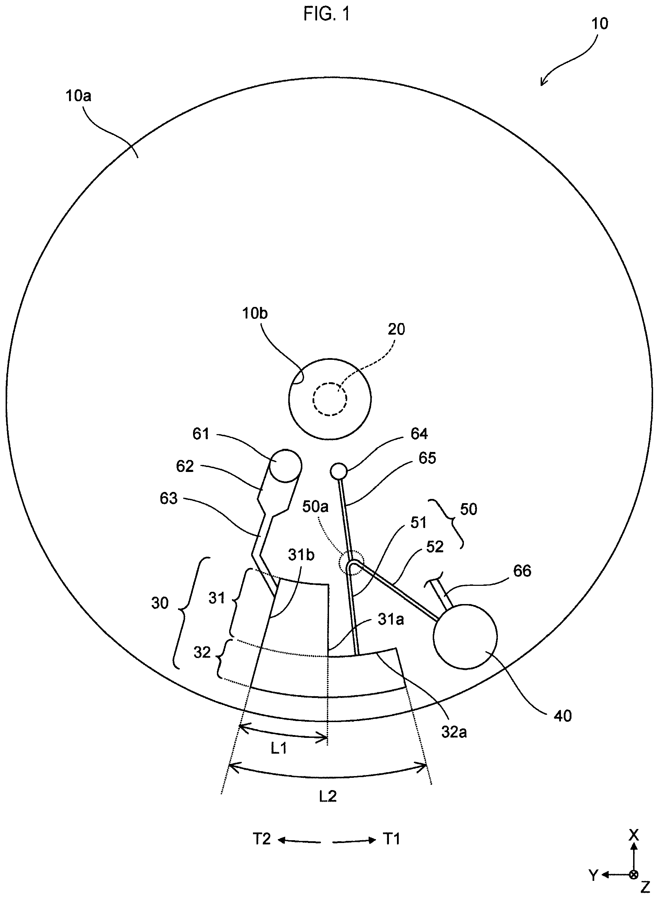

As shown in FIG. 1, the measuring cartridge 10 is a measuring cartridge for separating a liquid component from a sample by centrifugal separation, and subjecting the liquid component to measurement. The measuring cartridge 10 is a replaceable part that consolidates functions necessary for separating a liquid component from a sample by centrifugal separation. The measuring cartridge 10 is mounted on the measuring device so as to be rotatable around the rotating shaft 20 of the measuring device, and is configured to be capable of separating the sample accommodated therein into a solid component and a liquid component by centrifugal force. The measuring device rotates the rotating shaft 20 to rotate the mounted measuring cartridge 10 around the rotating shaft 20.

In the first embodiment, the sample is a blood sample of whole blood collected from the donor. The liquid component is a plasma component contained in a blood sample of whole blood. The solid component is a blood cell component contained in a blood sample of whole blood. Note that the sample is not limited to a blood sample of whole blood, inasmuch as the sample is collected from a subject. The liquid component is not limited to the plasma component and may be any liquid component contained in the sample collected from the subject. The solid component is not limited to the blood cell component and may be any solid component contained in the sample collected from the subject.

FIG. 1 is a schematic view of the measuring cartridge 10 mounted on the measuring device viewed in the vertical downward direction. In FIG. 1, the XYZ axes are orthogonal to each other. The X-axis positive direction indicates the backward direction, the Y-axis positive direction indicates the left direction, and the Z-axis positive direction indicates the vertical downward direction. In the following drawings, the XYZ axes also are the same as the XYZ axes in FIG. 1. Hereinafter, the radial direction of a circle centered on the rotating shaft 20 is referred to simply as the "radial direction". The circumferential direction of a circle centered on the rotating shaft 20, that is, the rotating direction around the rotating shaft 20, is referred to simply as the "rotation direction". In the rotation direction, the counterclockwise rotation as viewed in the Z axis positive direction is set as the T1 direction, and the clockwise direction as viewed in the Z axis positive direction is set as the T2 direction.

As shown in FIG. 1, the measuring cartridge 10 is configured by a plate-like and disk-shaped substrate 10a. Each part in the measuring cartridge 10 is formed by adhering a concave part in the substrate 10a and a film (not shown) covering the substrate 10a. The measuring cartridge 10 is not limited to being in the form of a plate, and may include a protruding part or the like, and is not limited to a disk shape and may be in other shapes such as a rectangular shape. The substrate 10a is provided with a hole 10b penetrating the substrate 10a at the center of the substrate 10a. The measuring cartridge 10 is installed in the measuring device so that the center of the hole 10b coincides with the rotating shaft 20 of the measuring device.

The measuring cartridge 10 includes a separation chamber 30, a receiving chamber 40, a flow path 50, a sample input port 61, a sample storage part 62, a flow path 63, a hole 64, an air introduction path 65, and a flow path 66.

The sample input port 61 is provided radially inner side of the sample storage part 62, and opens the inner side in the radial direction of the sample storage part 62 to the outside of the measuring cartridge 10. The sample storage part 62 accommodates the sample introduced from the sample input port 61. The flow path 63 is provided radially outer side of the sample storage part 62, and connects the sample storage part 62 and the separation chamber 30.

The separation chamber 30 has a first storage part 31 and a second storage part 32 arranged in a direction away from the rotating shaft 20 with respect to the first storage part 31. The second storage part 32 is connected to the first storage part 31. The first storage part 31 extends toward the rotating shaft 20, and the second storage part 32 extends along the rotation direction. The width L2 of the second storage part 32 in the rotation direction is larger than the width L1 of the first storage part 31 in the rotation direction. The first storage part 31 is disposed at a position biased in the T2 direction relative to the second storage part 32 in the rotation direction.

The first storage part 31 includes inner walls 31a and 31b. The inner walls 31a and 31b are parts of the first storage part 31 located on the opposite side of the rotating shaft 20 from the end part of the first storage part 31 positioned on the rotating shaft 20 side. The inner wall 31a is positioned on the T1 direction side of the first storage part 31 and the inner wall 31b is positioned on the T2 direction side of the first storage part 31. In plan view, the inner walls 31a, 31b extend in the radial direction. The inner wall 31b is connected to the inner wall on the T2 direction side of the second storage part 32 in the same plane. The flow path 63 is connected to the inner wall 31b. The second storage part 32 includes an inner wall 32a connected to the first storage part 31. The inner wall 32a is located at the end on the side of the rotary shaft 20 of the second storage part 32, and located on the T1 direction side of the first storage part 31. In a plan view, the inner wall 32a extends in the rotation direction. The flow path 50 is connected to the inner wall 32a.

The flow path 50 connects the separation chamber 30 and the receiving chamber 40. The flow path 50 extends in a direction from the separation chamber 30 toward the rotating shaft 20. Specifically, the flow path 51 of the flow path 50 connected to the separation chamber 30, which will be described later, extends in the direction toward the rotating shaft 20. Note that although the flow path 51 shown in FIG. 1 extends in the direction toward the rotating shaft 20, it suffices that the flow path 51 extends in the direction toward the rotating shaft 20, and need not necessarily be precisely directed toward the rotating shaft 20. That is, the flow path 51 does not necessarily extend in the radial direction. The flow path 50 also does not necessarily extend in the direction toward the rotating shaft 20 over the entire length, and may extend in the direction toward the rotating shaft 20 at least within a range in which the sample can enter before centrifugal separation.

The flow path 50 includes a flow path 51 and a flow path 52. The flow path 51 linearly extends from the separation chamber 30 toward the rotating shaft 20. The flow path 52 extends linearly in a direction away from the rotating shaft 20 from an end part of the flow path 51 on the side opposite the separation chamber 30. An end part of the flow path 51 on the T2 direction side is connected to the separation chamber 30, and an end part on the T1 direction side of the flow path 52 is connected to the receiving chamber 40. The end of the flow path 51 on the side opposite the separation chamber 30 and the end part of the flow path 52 on the side opposite the receiving chamber 40 are connected to each other at the connection position 50a. The flow path 50 is configured so that a liquid component separated in the separation chamber 30 moves from the separation chamber 30 toward the receiving chamber 40 through the flow path 50 by capillary action. Therefore, the inner diameter of the flow path 50 is set small enough to allow the liquid component to move by capillary action.

A hole 64 is provided on the inner side in the radial direction of the air introduction path 65, and opens the inner side in the radial direction of the air introduction path 65 to the outside of the measuring cartridge 10. The air introduction path 65 is connected to the connection position 50a of the flow path 50, and introduces air into the flow path 50 at the connection position 50a. Specifically, the air introduction path 65 introduces air into the flow path 51 from the connection position 50a, and introduces air into the flow path 52 from the connection position 50a. The receiving chamber 40 is a chamber for accommodating a liquid component separated by the separation chamber 30. The flow path 66 is connected to the receiving chamber 40. The liquid component transferred to the receiving chamber 40 via the flow path 50 is transferred to the another receiving chamber via the flow path 66 and the liquid component is measured in the other receiving chamber.

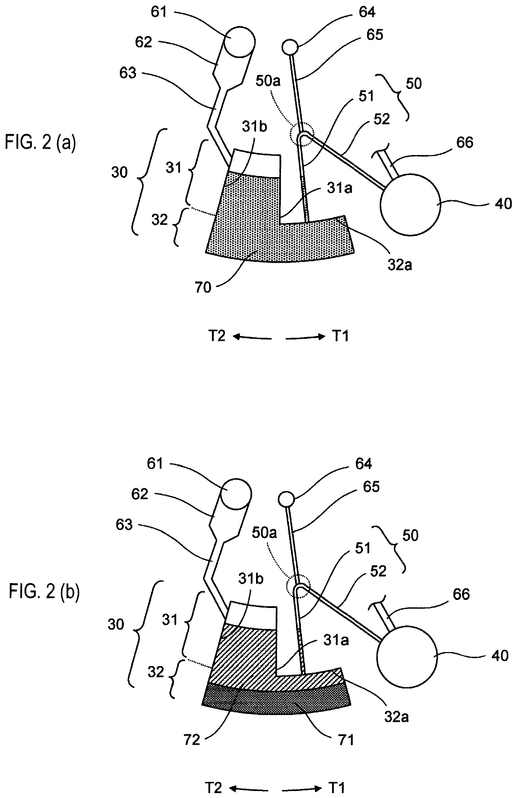

The procedure of separating the solid component 71 and the liquid component 72 contained in a sample 70, and accommodating the liquid component 72 in the receiving chamber 40 will be described below with reference to FIGS. 2 (a) to 3 (b).

The operator inserts the sample 70 into the sample input port 61 in advance and causes the sample storage part 62 to receive the sample 70. The operator mounts the measuring cartridge 10 in the measuring device and starts the operation by the measuring devices. The measuring device rotates the measuring cartridge 10 around the rotating shaft 20, and transfers the sample 70 accommodated in the sample storage part 62 to the separation chamber 30 via the flow path 63 by centrifugal force. At this time, as shown in FIG. 2 (a), the interface between the sample 70 and the air layer in the separation chamber 30 is located radially inner side of the position where the flow path 51 is connected to the inner wall 32a of the separation chamber 30. In this way, the sample 70 enters the vicinity of the end part of the flow path 51 on the separation chamber 30 side.

Subsequently, the measuring device rotates the measuring cartridge 10 around the rotating shaft 20 from the state shown in FIG. 2 (a), and centrifugally moves the sample 70 accommodated in the separation chamber 30 by rotation around the rotating shaft 20 so that the sample 70 is separated into a solid component 71 and a liquid component 72. In this way, as shown in FIG. 2 (b), in the separation chamber 30, the solid component 71 moves radially outward, and the liquid component 72 moves radially inward. In this case, since the flow path 50 extends from the separation chamber 30 in the direction toward the rotating shaft 20, as shown in FIG. 2 (a), the solid component 71 entering the flow path 50 from the separation chamber 30 before centrifugal separation moves from the flow path 50 to the separation chamber 30 by centrifugal force during centrifugal separation. Therefore, it is possible to prevent the solid component 71 from remaining in the flow path 50 after centrifugal separation.

It also is possible to extend the flow path 51 in the radial direction from the inner wall 32a since the flow path 51 is connected to the inner wall 32a. As shown in FIG. 1, the flow path 51 of the first embodiment extends radially from the inner wall 32a. In this way, the solid component 71 can enter the flow path 51 from the separation chamber 30 before centrifugation and accumulate in the flow path 51, then moves more smoothly to the first storage part 31 by centrifugal force during centrifugation. Therefore, it is possible to more reliably prevent the solid component 71 from remaining in the flow path 50 after centrifugal separation.

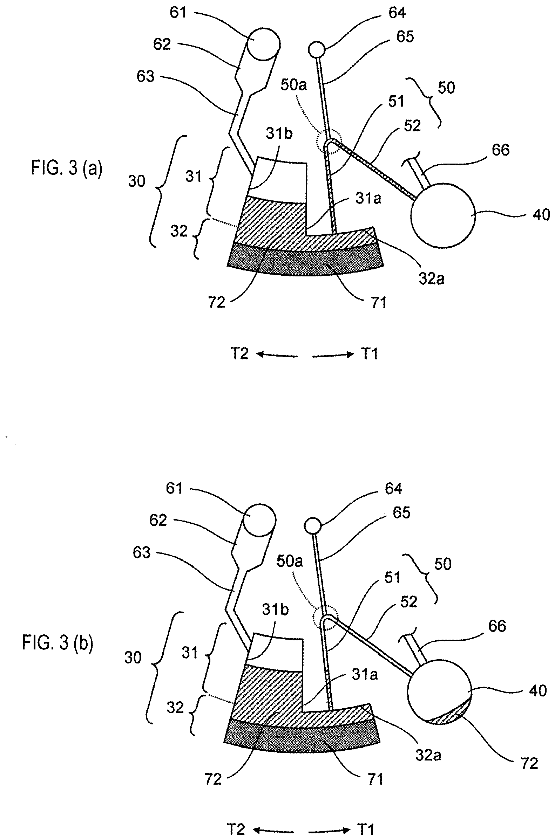

Subsequently, the measuring device stands by for a predetermined time without rotating the measuring cartridge 10 from the state of FIG. 2 (b). In this way, as shown in FIG. 3 (a), the liquid component 72 in the separation chamber 30 enters the flow path 50 by capillary action, and the inside of the flow path 50 is filled with the liquid component 72. Here, as described above, since the length of the first storage 31 is shorter than the length of the second storage part 32 in the rotation direction, the interface between the air layer and the liquid component 72 appearing in the first storage part 31 after the centrifugation is much farther from the second storage part 32 toward the rotating shaft 20 side. Therefore, the distance between this interface and the liquid layer of the solid component 71 after centrifugal separation can be increased, and the flow path 50 can be connected at a position distant from the liquid layer of the solid component 71. Specifically, as in the first embodiment, the flow path 50 can be connected to the inner wall 32a of the second storage part 32. In this way, it is possible to prevent the centrifugally separated solid component 71 from being caught in the flow generated by the capillary phenomenon.

As described above according to the first embodiment, it is possible to prevent the solid component 71 from remaining in the flow path 50 after centrifugal separation, and to prevent the centrifugally separated solid component 71 from being caught in the flow path 50. In this way, it is possible to prevent the solid component 71 from being mixed in the liquid component 72 moving in the flow path 50 from the separation chamber 30 toward the receiving chamber 40 through capillary action. Hence, it is possible to improve measurement accuracy of the liquid component 72 performed in the latter stage of the receiving chamber 40.

Subsequently, the measuring device rotates the measuring cartridge 10 around the rotating shaft 20 from the state of FIG. 3 (a). In this way as shown in FIG. 3 (b), a centrifugal force is applied to the liquid component 72 in the flow path 50, and the liquid component 72 in the flow path 51 is returned to the separation chamber 30 by centrifugal force, and the liquid component 72 in the flow path 52 is moved to the receiving chamber 40 by centrifugal force. At this time, air enters the flow path 50 from the air introduction path 65, the liquid component 72 that filled the flow path 50 from within the flow path 50 is separated at the connection position 50a such that the liquid component 72 is smoothly transported. In other words, the flow path 50 introduces air from the connection position 50a into the flow path 50, transfers the liquid component 72 that filled between the connection position 50a and the separation chamber 30 into the separation chamber 30, and transfers the liquid component 72 that filled between the container 50a and the receiving chamber 40 into the receiving chamber 40. Since the liquid component 72moving to the receiving chamber 40 becomes the liquid component 72 that fills the flow path 52, the quantitativeness of the liquid component 72 moving to the receiving chamber 40 is improved. That is, it is possible to transport the liquid component 72 in an amount necessary for measurement to the receiving chamber 40 without excess or deficiency.

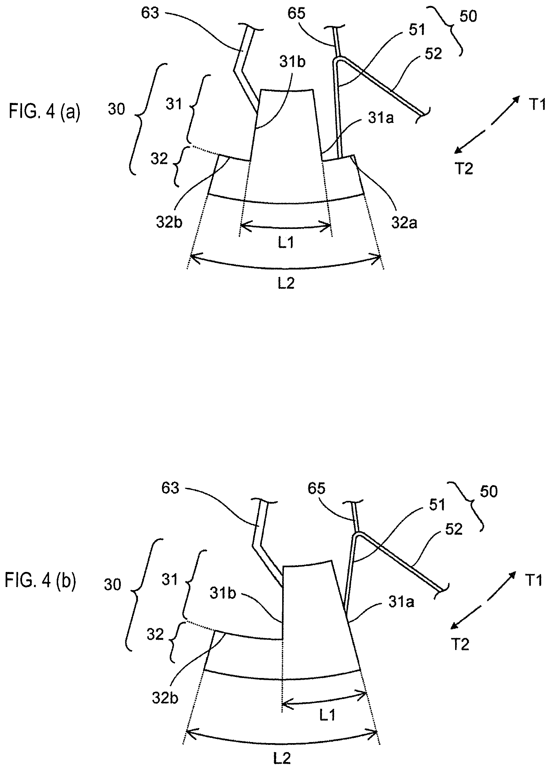

Note that, as shown in FIG. 1, the first storage part 31 of the first embodiment was disposed at a position biased in the T2 direction relative to the second storage part 32 in the rotation direction. However, the present invention is not limited to this arrangement inasmuch as the first storage part 31 may be at a position deviated in the T1 direction relative to the second storage part 32, as shown in Fig. 4 (b). Fig. 4 (a) shows an embodiment not covered by the present invention, where the first storage part 31 is disposed in the vicinity of the center position of the second storage part 32.

When the first storage part 31 is disposed as shown in FIG. 4 (a), in addition to the inner wall 32a, the second storage part 32 is provided with an inner wall 32b connected to the first storage part 31 and positioned on the T2 direction side of the first storage part 31. In plan view, the inner wall 32b extends in the rotation direction. When the first storage part 31 is arranged as shown in FIG. 4 (b), the second storage part 32 includes only the inner wall 32b among the inner walls 32a and 32b, as compared with FIG. 4 (a). In this case, the inner wall 31 a of the first storage part 31 is connected to the inner wall of the second storage part 32 on the T1 direction side in the same plane. The flow path 50 is connected to the inner wall 31a.

The flow path 50 is not limited to being connected to the inner wall 32a as shown in FIG. 1 and FIG. 4 (a), in as much as the flow path 50 also may be connected to the part of the second storage part 32 positioned on the rotating shaft 20 side from the end part of the second storage part 32 disposed on the side opposite the rotating shaft 20. Specifically, the flow path 50 may be connected to the inner wall 32b in FIGS. 4 (a) and 4 (b), and the flow path 50 also may be connected to the inner wall on the T1 direction side or the T2 direction side of the second storage part 32 in FIGS. 1 and 4 (a).

The flow path 50 is not limited to being connected to the inner wall 31a as shown in FIG. 4 (b), and also may be connected to part of the first storage part 31 positioned on the opposite side relative to rotating shaft 20 from the end part of the first storage part 31 located on the rotation shaft 20 side. Specifically, the flow path 50 may be connected to the inner wall 31a in FIGS. 1 and 4 (a), or may be connected to the inner wall 31b in FIG. 1 and FIGS. 4 (a) and 4 (b).

The flow path 50 also may be connected to the separation chamber 30 at the connection between the inner wall 31a and the inner wall 32b. The flow path 50 also may be connected to the separation chamber 30 at the connection between the inner wall 31b and the inner wall 32b. The flow path 50 also may be connected to the separation chamber 30 at a connection part between the first storage part 31 and the second storage part 32 in a plane including the inner wall 31b, as shown in FIG. 1. The flow path 50 also may be connected to the separation chamber 30 at a connection part between the first storage part 31 and the second storage part 32 in a plane including the inner wall 31a shown in FIG. 4 (b).

In the configurations shown in FIGS. 1 and 4 (a) and 4 (b), the flow path 63 was connected to the separation chamber 30 on the inner wall 31b of the first storage part 31. However, the flow path 63 is not limited to this arrangement, and may be connected to the separation chamber 30 on the inner wall 31a of the first storage part 31 or to the separation chamber 30 on the inner wall of the first storage part 31 located on the rotation shaft 20 side.

### Second Embodiment

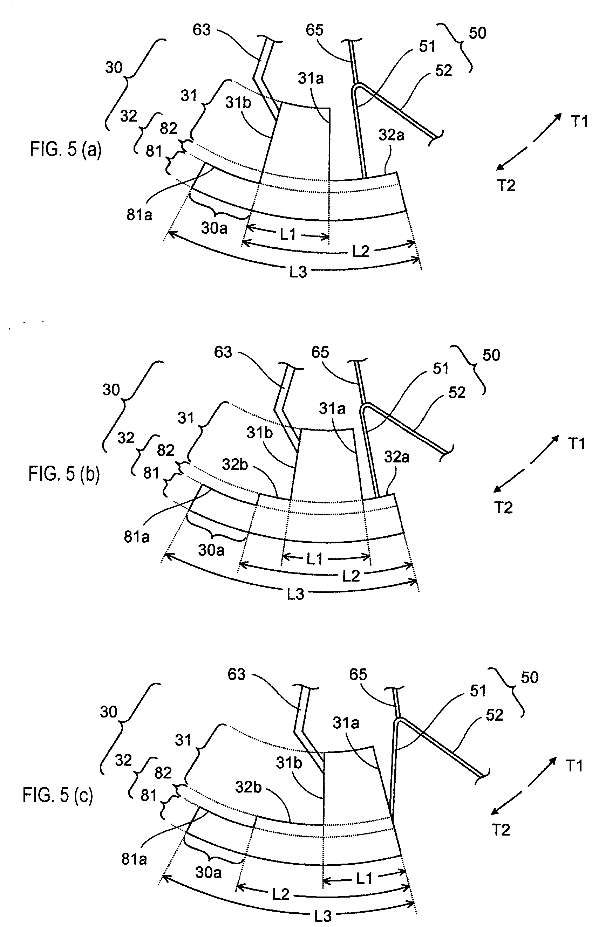

In the second embodiment shown in FIG. 5 (a), the separation chamber 30 also is provided a protruding part 30a that projects the inner wall part on the radial direction side of the second storage part 32 in the T2 direction in comparison with the configuration of the first embodiment shown in FIG. 1. In other words, the second storage part 32 of the second embodiment includes a first area 81, and a second area 82 arranged on the rotation shaft 20 side relative to the first area 81. The width L2 of the second area 82 in the rotation direction is smaller than the width L3 of the first area 81 in the rotation direction. The second area 82 is disposed at a position biased in the T1 direction relative to the first area 81. The first area 81 includes an inner wall 81a extending along the rotation direction in plan view. The inner wall 81a is positioned on the T2 direction side in the first area 81 and is connected to the second area 82. The inner wall 81a is located at the end of the first area 81 on the rotation shaft 20 side.

When the first area 81 and the second area 82 are formed in this manner, the solid component 71 can be efficiently accommodated in the wide first area 81 during centrifugal separation. As compared with the first embodiment, the interface between the specimen 70 and the air layer can be positioned radially outwardly in the separation chamber 30 by providing the protruding part 30a. In this way, the sample 70 can be centrifuged in a short time. The liquid layer of the solid component 71 also is moved away from the rotating shaft 20 by providing the protruding part 30a, compared with the first embodiment. In this way, it is possible to prevent the solid component 71 from getting caught in the flow to the flow path 50 due to capillary action.

Since the width L2 of the second area 82 is smaller than the width L3 of the first area 81, the interface between the air layer and the liquid component 72 after the centrifugal separation is located closer to the rotary shaft 20 side compared with the case where the entire inner wall on the T2 direction side of the second storage part 32 protrudes in the T2 direction from the configuration of FIG. 1. In this case, since the connection position of the flow path 50 relative to the separation chamber 30 can be brought close to the rotating shaft 20, the connection position of the flow path 50 is far from the liquid layer of the solid component 71. In this way, it is possible to more reliably prevent the solid component 71 from being caught in the flow to the flow path 50 through capillary action.

The first storage part 31 of the second embodiment is disposed at a position deviated to the T2 direction side relative to the second area 82, and the flow path 50 is disposed at a position of the second area 82 on the T1 direction side relative to the first storage part 81. When the flow path 50 is connected to the inner wall 32a in this manner, it is possible to prevent the liquid component 72 from moving away from the connection position on the inner wall 32a of the flow path 50 while the liquid component 72 flows into the flow path 50 via capillary action. In this way, the liquid component 72 can be stably supplied to the flow path 50 while the liquid component 72 flows into the flow path 50 due to capillary action. Note that the manner in which the interface between the air layer and the liquid component 72 advances in this case will be described in detail in a specific configuration example described later.

As shown in FIG. 5 (a), the separation chamber 30 of the second embodiment provides a protruding part 30a on the inner wall on the T2 direction side of the second storage part 32 in the configuration shown in FIG. 1. However, the invention is not limited to this arrangement inasmuch as the separation chamber 30 also may be configured by providing the protrusion 30a similar to FIG. 5 (a) in the configuration shown in FIG. 4 (a) as shown in FIG. 5 (b). As shown in FIG. 5 (c), the separation chamber 30 also may be configured by providing the protrusion 30a similar to FIG. 5 (a) in the configuration shown in FIG. 4 (b). In the case of the configuration shown in FIG. 5 (c), for example, the flow path 51 is connected to the border position between the first storage part 31 and the second storage part 32.

### Third Embodiment

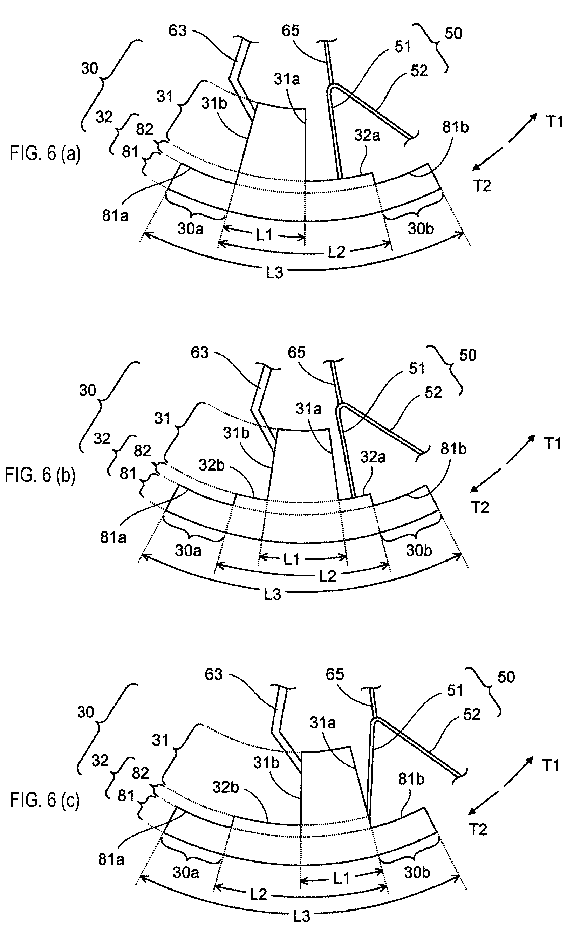

In the third embodiment shown in FIG. 6 (a), the separation chamber 30 is provided with a protruding part 30b that projects the inner wall part to the outer side in the radial direction among the inner wall on the T1 side of the second storage part 32, in comparison with the configuration of the second embodiment shown in FIG. 5 (a). In other words, the first area 81 of the third embodiment has a shape extending in the T1 direction as compared with the second embodiment. The first area 81 further includes an inner wall 81b extending along the rotation direction in plan view. The inner wall 81b is positioned on the T1 direction side and the radial direction inner side in the first area 81, and is connected to the second area 82.

In the third embodiment, the width L3 of the first area 81 is longer than the width L3 of the second embodiment. In this way, the solid component 71 can be accommodated more efficiently in the first area 81 during centrifugal separation. The interface between the sample 70 and the air layer also can be positioned radially outwardly in the separation chamber 30 by providing the protruding part 30b, as compared with the second embodiment. In this way, the sample 70 can be centrifuged in a shorter time. The liquid layer of the solid component 71 also is further away from the rotating shaft 20 by providing the protruding portion 30 b, as compared with the second embodiment. In this way, it is possible to further suppress the solid component 71 from getting caught in the flow to the flow path 50 through capillary action.

Note that, in the separation chamber 30 of the third embodiment shown in FIG. 6 (a), a protruding part 30b is provided on the inner wall on the T1 direction side of the second storage part 32 in the configuration shown in FIG. 5 (a). However, the cartridge is not limited to this arrangement inasmuch as shown in FIG. 6 (b), the separation chamber 30 also may provide the protrusion 30b similar to FIG. 6 (a) in the configuration shown in FIG. 5 (b), as shown in FIG. 6 (b). The separation chamber 30 also may provide the protruding part 30b similar to FIG. 6 (a) in the configuration shown in FIG. 5 (c), as shown in the configuration of FIG. 6 (c).

### Specific Structural Examples

Specific configurations of the measuring device and a specific configuration of the measuring cartridge of the second embodiment shown in FIG. 5 (a) will be described below. Note that in the following description the same configuration as that of the measuring cartridge 10 of the second embodiment explained with reference to FIG. 5 (a) is omitted for the sake of convenience.

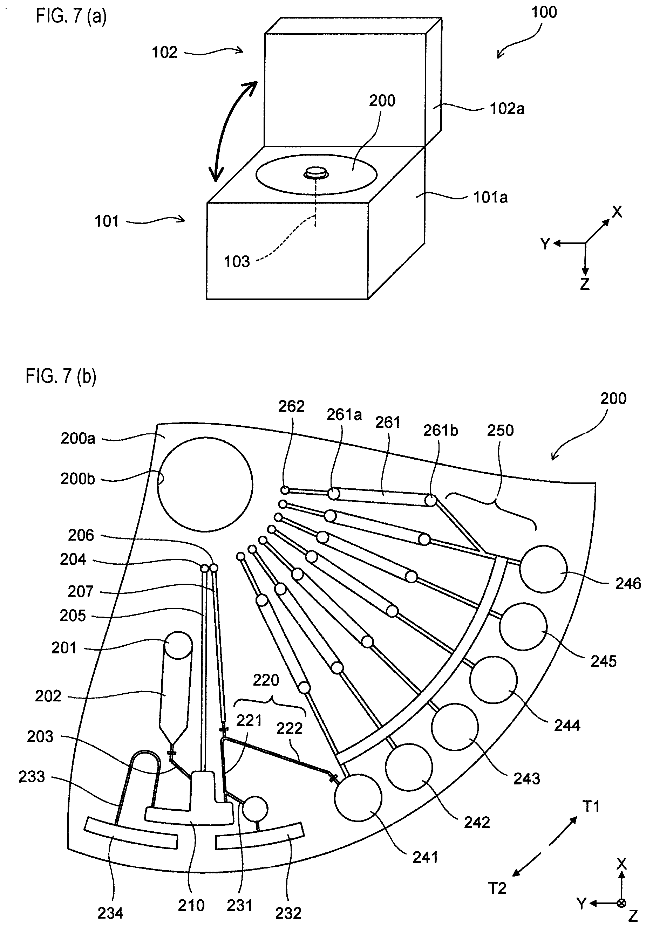

As shown in FIG. 7 (a), the measuring device 100 uses a measuring cartridge 200 to separate a liquid component from a sample, detect a target substance in the liquid component by utilizing antigen-antibody reaction, and analyze the target substance based on the detection result. Also in the specific configuration example, the sample is a blood sample of whole blood collected from a donor. The liquid component is a plasma component contained in a blood sample of whole blood. The solid component is a blood cell component contained in a blood sample of whole blood.

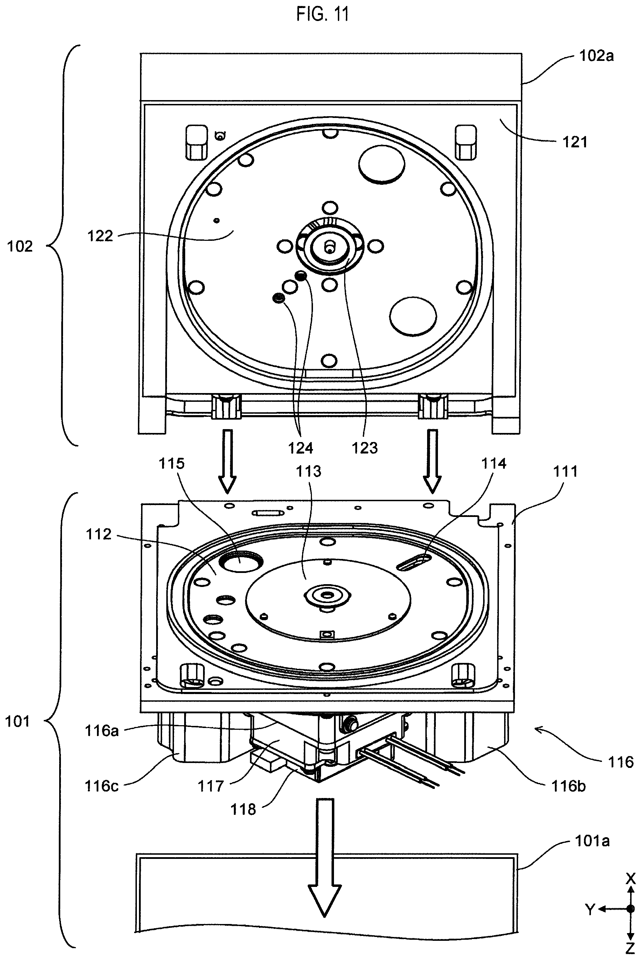

The measuring device 100 includes a main body 101 and a lid 102. In the main body 101, the part other than a part facing the lid 102 is covered with the housing 101a. In the lid 102, the part other than the part facing the main body 101 is covered with the housing 102a. The main body 101 supports the lid 102 so as to be openable and closable. When attaching and detaching the measuring cartridge 200, the lid 102 is opened as shown in FIG. 7 (a). The measuring cartridge 200 is mounted on the upper part of the main body 101. The main body 101 also includes a rotating shaft 103 extending parallel to the Z axis direction. The rotating shaft 103 corresponds to the rotating shaft 20 in FIG. 1. The measuring device 100 rotates the attached measuring cartridge 200 around the rotating shaft 103. The internal configuration of the measuring device 100 will be described later with reference to FIGS. 11 to 13.

As shown in FIG. 7 (b), the measuring cartridge 200 corresponds to the measuring cartridge 10 of the second embodiment shown in FIG. 5 (a). The measuring cartridge 200 is configured by a plate-like and disk-shaped substrate 200a. FIG. 5 (a) shows a part of the measuring cartridge 200. Each part in the measuring cartridge 200 is formed by adhering a concave part formed in the substrate 200a and a film (not shown) covering the substrate 200a. The substrate 200a and the film adhered to the substrate 200a are made of a light-transmitting member. The thickness of the substrate 200a is, for example, several millimeters, specifically 1.2 mm. The substrate 200a is provided with a hole 200b penetrating the substrate 200a at the center of the substrate 200a. The measuring cartridge 200 is installed in the measuring device 100 so that the center of the hole 200b coincides with the rotating shaft 103 of the measuring device 100.

The measuring cartridge 200 includes a sample input port 201, a sample storage part 202, a flow path 203, holes 204 and 206, air introduction paths 205 and 207, valves 208a, 208b and 208c, a separation chamber 210, a flow path 220, a flow path 231, an overflow chamber 232, a flow path 233, a waste chamber 234, receiving chambers 241 to 246, a flow path 250, a liquid storage part 261, and a hole 262.

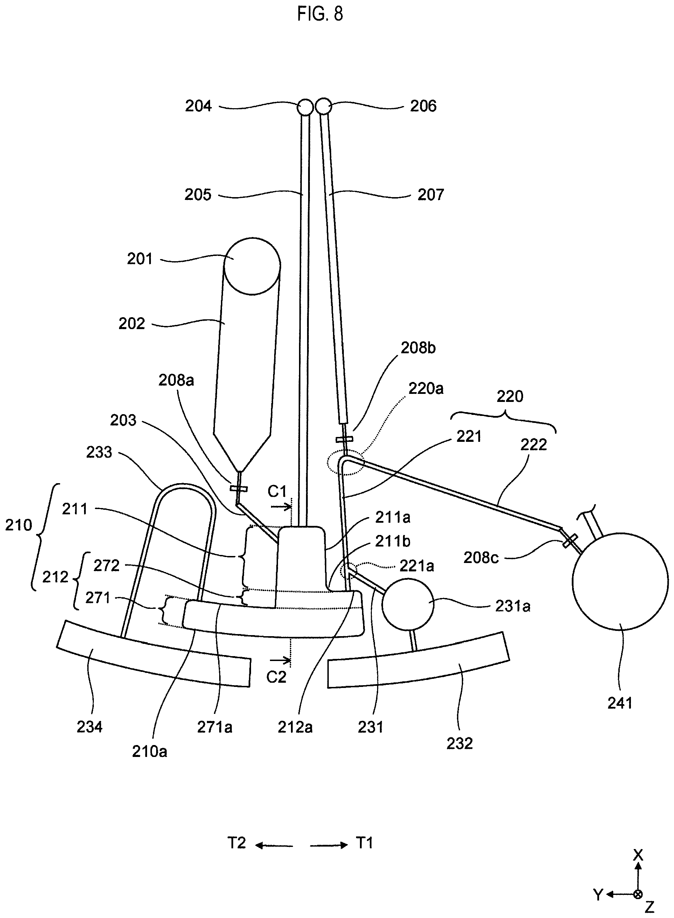

As shown in FIG. 8, the sample input port 201, the sample storage part 202, and the flow path 203 correspond to the sample input port 61, the sample storage part 62, and the flow path 63 shown in FIG. 1, respectively. The valve 208a is provided between the sample storage part 62 and the flow path 203. Before the measuring cartridge 200 is rotated, the valve 208a restrains the sample accommodated in the sample storage part 202 from moving to the flow path 203.

The separation chamber 210 includes a first storage part 211 and a second storage part 212, and the second storage part 212 includes a first area 271 and a second area 272. The separation chamber 210 corresponds to the separation chamber 30 shown in FIG. 5 (a), and the first storage part 211 and the second storage part 212 correspond to the first storage part 31 and the second storage part 32, and the first area 271 and the second area 272 correspond to the first area 81 and the second area 82 shown in FIG. 5 (a), respectively.

The first storage part 211 includes an inner wall 211a, and the second storage part 212 includes an inner wall 212a. The inner walls 211a and 212a correspond to the inner walls 31a and 32a shown in FIG. 5 (a), respectively. The inner wall 211a is connected to the inner wall 21 a by a curved inner wall 211b which is inclined so as to be gradually parallel to the inner wall 212a from an end edge on the inner wall 212a side of the inner wall 211a, and is connected to the inner wall 212a. That is, the inner wall 211a extending in the radial direction in plan view and the inner wall 212a extending in the rotation direction in plan view are smoothly connected by the curved inner wall 211b.

The first region 271 includes an inner wall 271a. The inner wall 271a corresponds to the inner wall 81a shown in FIG. 5 (a). The width in the rotation direction of the inner wall 271a is larger than the width in the rotation direction of the inner wall 212a. The protruding portion 210a is a part of the first area 271 protruding in the T2 direction relative to the second area 272. The protruding part 210a corresponds to the protruding part 30a shown in FIG. 5 (a).

An air introduction path 205 is connected to the inner wall of the separation chamber 30 extending in the radial direction on the inner side in plan view. The hole 204 is provided radially inward of the air introduction path 205, and opens the inside of the air introduction path 205 in the radial direction to the outside of the measuring cartridge 200.

An air introduction path 207 is connected to the connection position 220a of the flow path 220. The hole 206 is provided radially inward of the air introduction path 207, and opens the inside of the air introduction path 207 in the radial direction to the outside of the measuring cartridge 200. The air introduction path 207 introduces air into the flow path 220 at the connection position 220a. The hole 206, the air introduction path 207, and the connection position 220a correspond to the hole 64, the air introduction path 65, and the connection position 50a shown in FIG. 1, respectively. The valve 208b is provided between the air introduction path 207 and the connection position 220a. The valve 208b prevents the liquid component that enters the flow path 220 through capillary action from entering the air introduction path 207.

The flow path 220 includes a flow path 221 and a flow path 222. The flow path 220 corresponds to the flow path 50 shown in FIG. 5 (a), and the flow path 221 and the flow path 222 correspond to the flow path 51 and the flow path 52 shown in FIG. 5 (a). The valve 208c is provided between the flow path 222 and the storage chamber 241 on the side of the storage chamber 241 of the flow path 222. The valve 208c is provided to stop the movement of the liquid component through capillary action. That is, the valve 208c prevents the liquid component that enters the flow path 220 from the separation chamber 210 via capillary action from entering the receiving chamber 241.

The flow path 231 connects the flow path 221 and the overflow chamber 232. Specifically, the end of the flow path 231 on the flow path 221 side is connected to the branching position 221a that is closer to the separation chamber 210 than the center of the flow path 221. The flow path 231 is provided with a storage part 231a. The overflow chamber 232 contains unnecessary analytes and unnecessary liquid component. In other words, the overflow chamber 232 is provided for discarding unnecessary sample and unnecessary liquid component.

The flow path 233 connects the first area 271 and the waste chamber 234. Specifically, one end part of the flow path 233 is connected to the inner wall 271a of the first area 271. The flow path 233 moves the liquid component remaining in the separation chamber 210, after the liquid component is moved from the separation chamber 210 to the storage chamber 241, from the first area 271 to the waste chamber 234 according to the siphon principle. Waste chamber 234 contains unnecessary liquid component. That is, the waste chamber 234 is provided for discarding unnecessary liquid component.

Returning to FIG. 7 (b), the receiving chambers 241 to 246 are arranged in the rotation direction near the outer periphery of the substrate 200a. The receiving chamber 241 corresponds to the receiving chamber 40 shown in FIG. 1. The flow path 250 includes an arcuate region extending in the rotation direction, and a region for moving the reagent in the liquid storage part 261 toward the corresponding receiving chamber. The liquid storage part 261 contains reagents and includes sealing bodies 261a and 261b. The sealing bodies 261a, 261b are configured to be able to be opened by being pushed from above by a pressing part 124 described later. When the sealing bodies 261a and 261b are opened, the inner side in the radial direction of the liquid storage part 261 is opened to the outside of the measuring cartridge 200 via the hole 262, and the outer side in the radial direction of the liquid storage part 261 is connected to the flow passage 250. In this way, the reagent in the liquid storage part 261 can be transferred to the corresponding receiving chamber among the receiving chambers 241 to 246 via the flow path 250 through centrifugal force.

Each configuration of the measuring cartridge 200 shown in FIG. 7 (b) is formed only in one third of the area of the substrate 200a. However, the present invention is not limited to this arrangement inasmuch as a group of these configurations may be formed in the remaining two-thirds region, and three groups of structures may be provided on the substrate 200a.

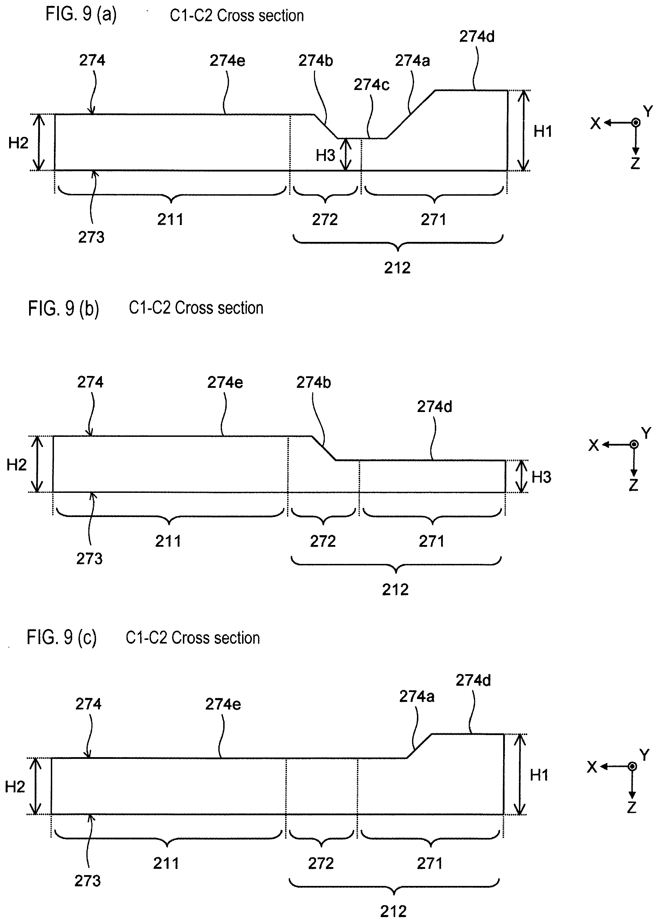

FIG. 9 (a) is a diagram schematically showing the configuration of the separation chamber 210 when the cross section of C1 - C2 shown in FIG. 8 is viewed in the Y axis negative direction. In FIG. 9 (a), the X-axis positive direction indicates a direction toward the rotation axis 103, and the X-axis negative direction indicates a direction away from the rotation axis 103.

As shown in FIG. 9 (a), the separation chamber 210 has a lower surface 273 as an inner wall located on the Z axis positive direction side, and an upper surface 274 as an inner wall located on the Z axis negative direction side. The lower surface 273 is a flat surface parallel to the XY plane. The upper surface 274 includes a first inclined part 274a, a second inclined part 274b, and flat parts 274c, 274d, and 274e.

The first inclined part 274a increases the thickness of the space in the second storage part 212 as the distance from the rotation shaft 103 increases. The first inclined part 274a is provided in the first area 271 of the separation chamber 210. The second inclined part 274b reduces the thickness of the space in the second storage part 212 as it moves away from the rotating shaft 103. The second inclined part 274b is provided in the second area 272 of the separation chamber 210. The flat part 274c is a flat surface parallel to the XY plane and connects the first inclined part 274a and the second inclined part 274b.

The flat part 274d is a flat surface parallel to the XY plane, and the thickness of the space in the second storage part 212 in the first rare 271 is defined as H1. The flat part 274e is a flat surface parallel to the XY plane, and the thickness of the space in the second storage part 212 in the second area 272, and the thickness of the space in the first storage part 211 are defined as H2. The flat part 274c regulates the thickness of the space in the second storage part 212 at the border between the first area 271 and the second area 272 to H3. The relationship between the thicknesses H1 to H3 is H1>H2>H3.

By providing the first inclined portion 274a, it is possible to increase the thickness of the area radially outside of the first inclined portion 274a to increase the capacity of the area radially outward of the first inclined portion 274a. In this way, it easier for the solid component to stay in an area radially outward of the first inclined part 274a during centrifugation due to the boycott effect, thereby increasing centrifugal separation efficiency. The solid component can be efficiently collected in the area radially outward of the first inclined part 274a since the liquid component is smoothly moved along the first inclined part 274a to the inner side in a radial direction from the outer side in the radial direction of the second storage part 212.

By providing the second inclined part 274b, it is possible to reduce the thickness of the area on the outer side in the radial direction by the second inclined part 274b to reduce the thickness of the area on the radially outer side from the second inclined part 274b. In this way, the solid component moved to the outer side in the radial direction from the second inclined part 274b by centrifugal force is unlikely to return to the radially inner side. Hence, mixing of the solid component into the flow path 220 after the centrifugal separation can be effectively prevented. The solid component can be smoothly moved outward in the radial direction of the second inclined part 274b along the second inclined part 274b during centrifugal separation.

A convex part is disposed between the first area 271 in which the solid component is stored and the second area 272 in which the liquid component is stored by providing the first inclined part 274a and the second inclined part 274b. Specifically, a flat part 274c protruding in a direction of reducing the thickness of the separation chamber 30 is provided between the first area 271 and the second area 272. In this way, it is possible to prevent the solid component from mixing into the liquid component more effectively.

The thickness of the space in the second storage part 212 in the first are 271 is HI, and the thickness of the space in the second storage part 212 in the second area 272 is H2 which is smaller than H1. In this way, the solid component can be efficiently retained in the first area 271.

The first inclined part 274a and the second inclined part 274b are connected by a flat part 274c. In this way, the solid component does not easily move in the range where the flat part 274c is provided, so that the solid component retained at the outer side in the radial direction from the first inclined part 274a by the centrifugal separation passes through the flat part 274c, and mixing with the liquid component is prevented. Hence, it is possible to more effectively prevent the solid component from being mixed in the liquid component taken into the flow path 220 by capillary action.

Note that the separation chamber 210 is not limited to being configured as shown in FIG. 9 (a), and may be configured as shown in FIGS. 9 (b) and 9 (c), for example. In the configuration shown in FIG. 9 (b), the first inclined part 274a and the flat part 274c are omitted on the upper surface 274 of the separation chamber 210, as compared with FIG. 9 (a). In this case, the effect of the second inclined part 274b also is obtained. In the configuration shown in FIG. 9 (c), the second inclined part 274b and the flat part 274c are omitted on the upper surface 274 of the separation chamber 210, as compared with FIG. 9 (a). In this case, the effect by the first inclined part 274a also is obtained.

In addition, in the configuration shown in FIG. 9 (a), the flat part 274c may be omitted, and the first inclined part 274a and the second inclined part 274b may be adjacent to each other. The configuration of the inclined part, the flat part and the like is not limited to being provided on the upper surface 274, and also may be provided on the lower surface 273, or may be provided on both the lower surface 273 and the upper surface 274. The inclined surface of the first inclined part 274a and the inclined surface of the second inclined part 274b need not necessarily have a flat surface as shown in FIG. 9 (a), and may have irregularities.

In the configuration shown in FIG. 9 (a), the first inclined part 274a may be omitted, and the flat part 274c and the flat part 274d may be connected by a flat surface parallel to the YZ plane. In the configuration shown in FIG. 9 (c), the first inclined part 274a may be omitted, and the flat part 274d and the flat part 274e may be connected by a flat surface parallel to the YZ plane. In the configuration shown in FIG. 9 (a), the second inclined part 274b may be omitted, and the flat part 274c and the flat part 274e may be connected by a flat surface parallel to the YZ plane. In the configuration shown in FIG. 9 (b), the second inclined part 274b may be omitted, and the flat part 274d and the flat part 274e may be connected by a flat surface parallel to the YZ plane.

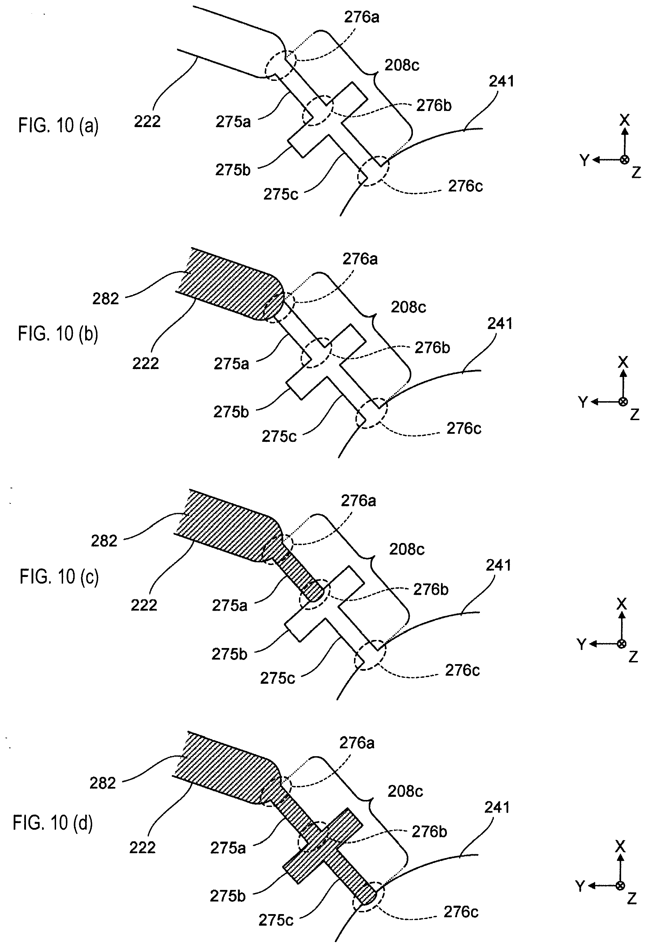

FIG. 10 (a) is an enlarged view schematically showing the vicinity of the valve 208c.

As shown in FIG. 10 (a), the valve 208c includes a flow path 275a, a space 275b, and a flow path 275c. The flow path 275a is connected to the flow path 222, and the flow path 275c is connected to the receiving chamber 241. The space 275b connects the flow path 275a and the flow path 275c.

The flow path 275a is configured so that the width of the flow path 275a sharply decreases as compared with the size of the flow path 222 in the connection part 276a between the flow path 222 and the flow path 275a. The space 275b is configured so that the space 275b abruptly increases in size at the connecting part 276b between the flow path 275a and the space 275b as compared with the size of the flow path 275a. The flow path 275c is configured such that the width of the flow path 275c is sharply smaller than the size of the receiving chamber 241 in the connection part 276c between the flow path 275c and the receiving chamber 241. The cross-sectional area of the flow paths 275a and 275c is constant. Note that the cross-sectional areas of the flow paths 275a and 275c need not necessarily be constant. The flow paths 275a, 275c, and the space 275b are configured to have low wettability with respect to liquid.

In the connecting part 276a, the width of the flow path 275a is abruptly smaller than the size of the flow path 222, and the flow path 275a has low wettability with respect to liquid. In this way, as shown in FIG. 10 (b), even if the liquid component 282 in the flow path 222 reaches the connecting part 276a due to capillary action, the liquid component 282 is unlikely to intrude into the flow path 275a. Hence, it is possible to prevent the liquid component 282 of the flow path 222 from entering the receiving chamber 241 through capillary action.

In general, the liquid component 282 in the flow path 222 does not enter the flow path 275a for the above-mentioned reason. However, the liquid component 282 in the flow path 222 may enter the flow path 275a due to capillary action. Therefore, the valve 208c is provided with a space 275b and a flow path 275c in addition to the flow path 275a.

In the connecting part 276b, the size of the space 275b is abruptly larger than the size of the flow path 275a, and the space 275b has low wettability with respect to liquid. In this way, as shown in FIG. 10 (c), even if the liquid component 282 reaches the connecting part 276b through capillary action, the liquid component 282 in the flow path 275a is unlikely to enter the space 275b due to the surface tension of the liquid component 282. Hence, it is possible to reliably prevent the liquid component 282 of the flow path 222 from entering the receiving chamber 241 through capillary action.

In the connecting part 276c, the size of the receiving chamber 241 becomes abruptly larger than the size of the flow path 275c. In this way, even if the liquid component 282 reaches the connecting part 276c through capillary action, the liquid component 282 in the flow path 275c is unlikely to intrude into the receiving chamber 241 due to the surface tension of the liquid component 282, as shown in FIG. 10 (d). Hence, it is possible to reliably prevent the liquid component 282 of the flow path 222 from entering the receiving chamber 241 through capillary action.

Note that the valve 208a also has flow paths 275a, 275c and a space 275b similar to the valve 208c. That is, the size of the flow path inside the valve 208a becomes abruptly smaller than the size of the sample storage part 202 and the flow path 203, and the size of the space within the valve 208a becomes abruptly larger compared to the size of the flow path inside the valve 208a. In this way, the valve 208a can prevent the sample in the sample storage part 202 from entering the flow path 203 through capillary action. Similarly to the valve 208c, the valve 208b also has flow paths 275a, 275c and a space 275b. That is, the size of the flow path inside the valve 208b becomes abruptly smaller than the size of the air introduction path 207 and the flow path 220, and the space in the valve 208b becomes abruptly larger compared to the size of the flow path inside the valve 208b. In this way, the valve 208b can prevent the liquid component in the flow path 220 from entering the air introduction path 207 through capillary action.

The internal configuration of the measuring device 100 will be described below referring to FIGS. 11 to 13.

The main body 101 includes a mounting member 111, a plate member 112, a support member 113, a magnetic force applicator 114, a detection unit 115, a housing body 116, a motor 117, and an encoder 118.

The mounting member 111 has a shape to be fitted into the casing 101a. The plate member 112 is installed at the center of the upper surface of the mounting member 111. The plate member 112 is made of a metal having high thermal conductivity. On the lower surface of the plate member 112, a heater 131 (described later) is installed. The support member 113 is installed at the center of the mounting member 111 via a mounting member 119 to be described later. The support member 113 is configured by, for example, a turn table.

The magnetic force applicator 114 is installed on the lower surface of the mounting member 111 so as to face the lower surface of the measuring cartridge 200 installed on the support member 113 via holes formed in the mounting member 111 and the plate member 112. The magnetic force applicator 114 includes a magnet and a mechanism for moving the magnet in the Z axis direction and the radial direction. The detection unit 115 is installed on the lower surface of the mounting member 111 so as to face the lower surface of the measuring cartridge 200 installed on the support member 113 via holes formed in the mounting member 111 and the plate member 112. The detection unit 115 includes a photodetector. The photodetector of the detection unit 115 optically detects the test substance contained in the receiving chamber 246. The photodetector of the detection unit 115 is composed of, for example, a photomultiplier tube, a photoelectric tube, a photodiode or the like.

The housing body 116 is installed on the lower surface of the mounting member 111. The housing body 116 includes a lower surface 116a and storage parts 116b and 116c. A hole 116d (described later) is formed at the center of the upper surface of the housing body 116. The hole 116d vertically penetrates from the upper surface of the housing body 116 to the lower surface 116a. A rotating shaft 103 passes through the hole 116d. The storage parts 116b and 116c are configured by concave parts recessed downward from the upper surface of the housing body 116. The storage parts 116b and 116c accommodate the magnetic force applying portion 114 and the detecting portion 115, respectively. The motor 117 is configured by a stepping motor. The motor 117 is installed on the lower surface 116a and rotates the rotating shaft 103 about the Z axis as the center of rotation. The encoder 118 is installed on the lower surface of the motor 117 and detects the rotation of a drive shaft 117a of the motor 117, which will be described later.

FIG. 11 also shows a state in which the lid 102 is viewed from below. The lid 102 includes a mounting member 121, a plate member 122, a clamper 123, and two pressing parts 124.

The mounting member 121 has a shape to fit in the housing body 102a. The plate member 122 is installed at the center of the lower surface of the mounting member 121. The plate member 122 is made of a metal having high thermal conductivity similar to the plate member 122. A heater 132 (described later) is installed on the upper surface of the plate member 122. The clamper 123 is installed at the center of the mounting member 121. The two pressing parts 124 are installed on the upper surface of the mounting member 121. When the lid 102 is closed, the two pressing parts 124 are arranged in the radial direction of the measuring cartridge 200 installed in the supporting member 113. The pressing part 124 on the inner side in the radial direction presses the sealing body 261a from above through the hole formed in the mounting member 121 and the plate member 122, and opens the sealing body 261a by a pressing force. The pressing part 124 on the outer side in the radial direction presses the sealing body 261b from the upper side through the hole formed in the mounting member 121 and the plate member 122, and opens the sealing body 261b by a pressing force.

During assembly of the measuring device 100, the mounting member 111 and the housing body 116 assembled as shown in FIG. 11 are installed in the housing body 101a to complete the main body 101. Then, as shown in FIG. 11, the lid 102 is installed in the main body part 101 by mounting the assembled lid 102 so as to be openable and closable relative to the mounting member 111 of the main body 101. In this way, the measuring device 100 is completed.

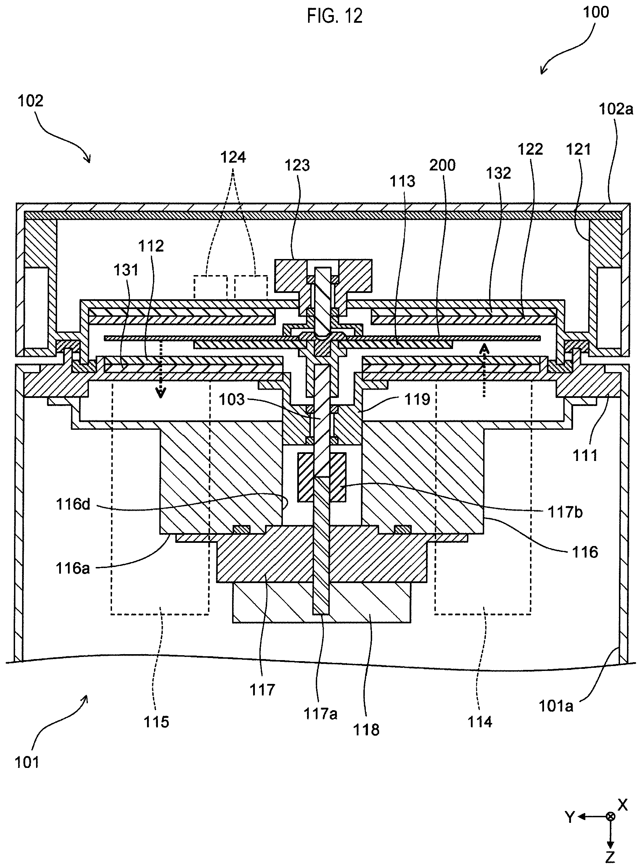

FIG. 12 is a schematic diagram showing a cross section of the measuring device 100 cut along a plane parallel to the YZ plane passing through the rotating shaft 103. FIG. 12 shows the state in which the measuring cartridge 200 is installed in the measuring device 100, and the lid 102 is closed. As described above, the magnetic force applicator 114 and the detection unit 115 are installed on the lower surface of the mounting member 111, and two pressing parts 124 are installed on the upper surface of the mounting member 121. In FIG. 12, positions corresponding to the arrangement positions of these parts are indicated by broken lines.

As shown in FIG. 12, the drive shaft 117a of the motor 117 extends inside the hole 116d. A mounting member 119 is installed above the hole 116d. The mounting member 119 rotatably supports the rotating shaft 103 extending in the vertical direction. The rotating shaft 103 is fixed to the drive shaft 117a of the motor 117 by a locking member 117b inside the hole 116d.

A support member 113 for supporting the lower surface of the measuring cartridge 200 is fixed to the upper part of the rotating shaft 103 via a predetermined member. When the motor 117 is driven and the drive shaft 117a rotates, the rotational driving force is transmitted to the support member 113 via the rotating shaft 103. In this way, the measuring cartridge 200 installed on the support member 113 rotates around the rotating shaft 103. When the measuring cartridge 200 is installed on the support member 113 and the lid 102 is closed, the clamper 123 presses the inner peripheral part of the upper surface of the measuring cartridge 200 in a rotatable state.

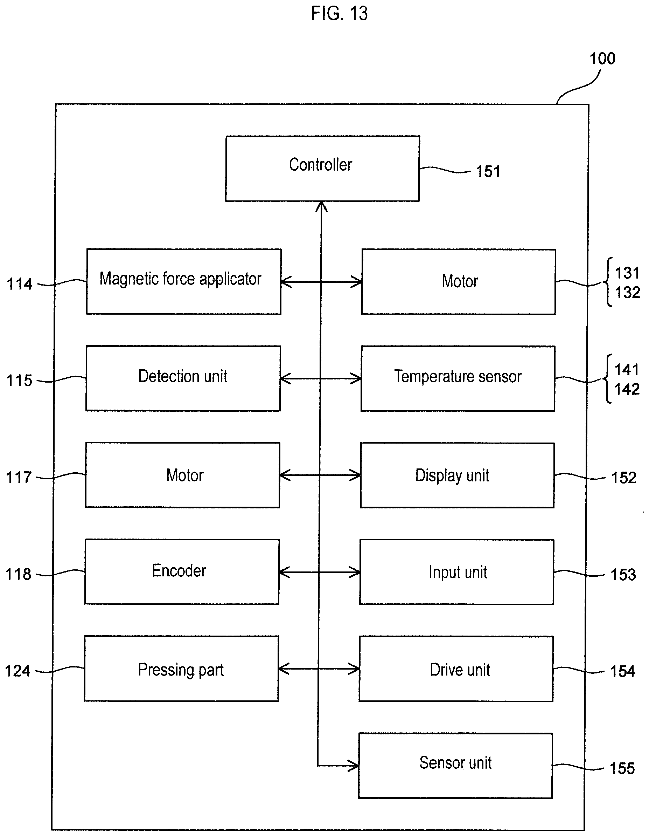

A heater 131 is installed on the lower surface of the plate member 112, and a heater 132 is installed on the upper surface of the plate member 122. The heaters 131 and 132 have a flat heat generation surface, and the heat generation surface is parallel to the measuring cartridge 200. In this way, the measuring cartridge 200 can be efficiently heated. Temperature sensors 141 and 142 shown in FIG. 13 are installed on the plate members 112 and 122, respectively. The temperature sensors 141 and 142 detect the temperatures of the plate members 112 and 122, respectively. At the time of measurement, a controller 151, which will be described later, drives the heaters 131 and 132 to heat the temperature of the plate member 112 detected by the temperature sensor 141 and the temperature of the plate member 122 detected by the temperature sensor 142 to predetermined temperature.

The magnetic force applicator 114 applies a magnetic force to the measuring cartridge 200 using a magnet as indicated by the upward dotted arrow in FIG. 12. The detection unit 115 receives light generated from the receiving chamber 246 of the measuring cartridge 200 as indicated by a downward dotted arrow in FIG. 12. When the lid 102 is closed, light is prevented from passing between the space where the measuring cartridge 200 is located and the outside. In this way, even if the light generated in the reaction process in the receiving chamber 246 is extremely weak, light generated by the reaction is detected by the photodetector of the detection unit 115 since light does not enter the space where the measuring cartridge 200 is located from the outside, and it becomes possible to detect with high accuracy.

As shown in FIG. 13, the measuring device 100 includes a magnetic force applicator 114, a detection unit 115, a motor 117, an encoder 118, a pressing part 124, heaters 131 and 132, temperature sensors 141 and 142, a controller 151, a display unit 152, an input unit 153, a drive unit 154, and a sensor unit 155.

The controller 151 includes, for example, an arithmetic processing unit and a storage unit. The arithmetic processing unit is configured by, for example, a CPU, an MPU or the like. The storage unit is composed of, for example, a flash memory, a hard disk or the like. The controller 151 receives signals from each unit of the measuring device 100 and controls each unit of the measuring device 100. The display unit 152 and the input unit 153 are provided, for example, on a side surface part of the main body 101, an upper surface part of the lid 102 or the like. The display unit 152 is configured by, for example, a liquid crystal panel. The input unit 153 is configured by, for example, a button, a touch panel or the like. The drive unit 154 includes another mechanism disposed in the measuring device 100. The sensor unit 155 includes a sensor for detecting a predetermined part of the measuring cartridge 200 mounted on the support member 113, and another sensor disposed in the measuring device 100.

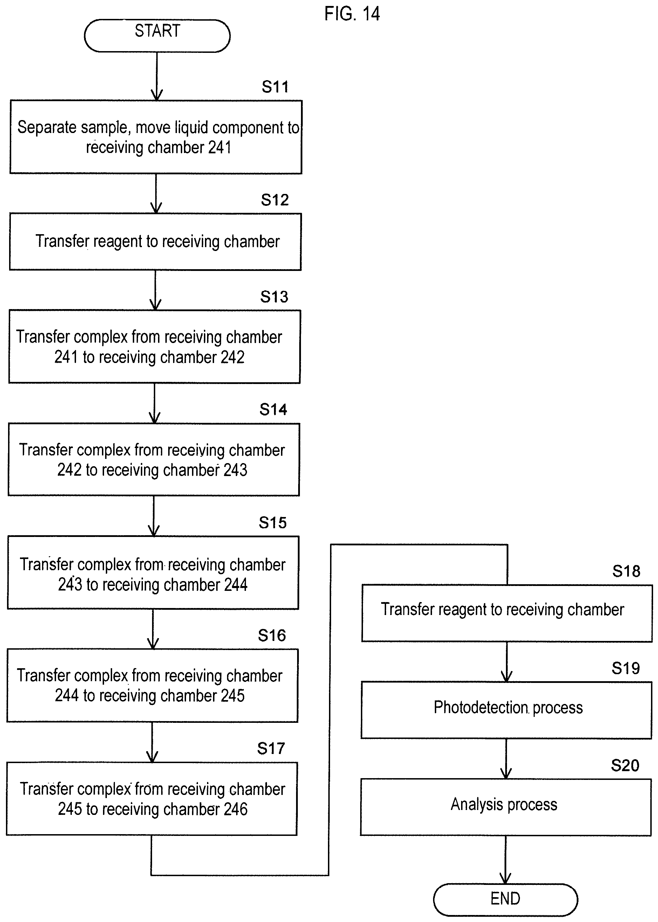

Next, the operation of the measuring device 100 will be described with reference to FIG. 14.

First, the operator inserts the sample collected from the donor through the sample input port 201, and places the measuring cartridge 200 on the support member 113. The sample inserted from the sample input port 201 is received in the sample storage part 202. The target substance in the sample contains, for example, an antigen. Such an example of the antigen is hepatitis B surface antigen (HBsAg). The target substance may be one or more of antigen, antibody, or protein.

Prescribed reagents are stored in advance in the seven liquid storage parts 261 and the receiving chamber 241 of the measuring cartridge 200. Specifically, the R1 reagent is contained in the liquid storage part 261 located in the radial direction of the receiving chamber 241. The R2 reagent is contained in the receiving chamber 241. The R3 reagent is contained in the liquid storage part 261 located in the radial direction of the receiving chamber 242. A cleaning liquid is contained in the liquid storage part 261 located in the radial direction of the receiving chambers 243 to 245. R4 reagent is contained in a liquid storage part 261 located in the radial direction of the receiving chamber 246. The R5 reagent is contained in the liquid storage part 261 adjacent to the T1 direction side of the liquid storage part 261 containing the R4 reagent.

In the following control, the controller 151 obtains the rotational position of the drive shaft 117a of the motor 117 based on the output signal of the encoder 118 connected to the motor 117. The controller 151 acquires the position in the rotation direction of the measuring cartridge 200 by detecting a predetermined part of the rotating measuring cartridge 200 with a sensor. Alternatively, the measuring cartridge 200 may be installed at a predetermined position with respect to the support member 113. In this way, the controller 151 can position each part of the measuring cartridge 200 at a predetermined position in the rotation direction.

Upon receiving a start instruction from the operator via the input unit 153, the controller 151 starts the process shown in FIG. 14. In step S11, the controller 151 separates the sample and transfers the liquid component to the receiving chamber 241.

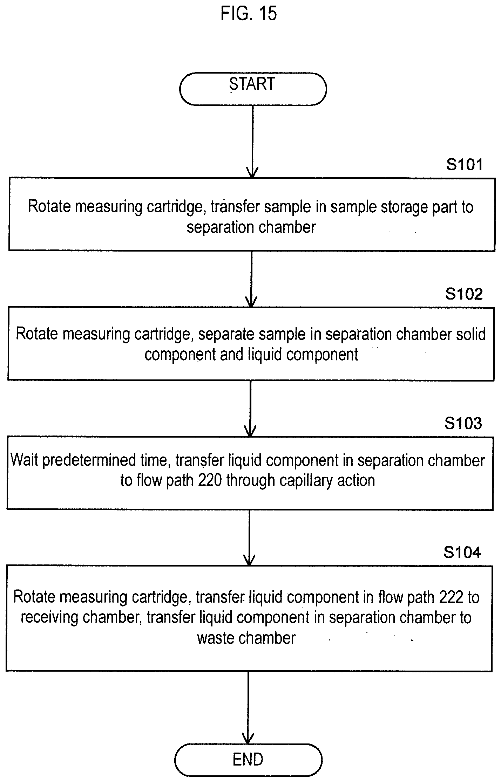

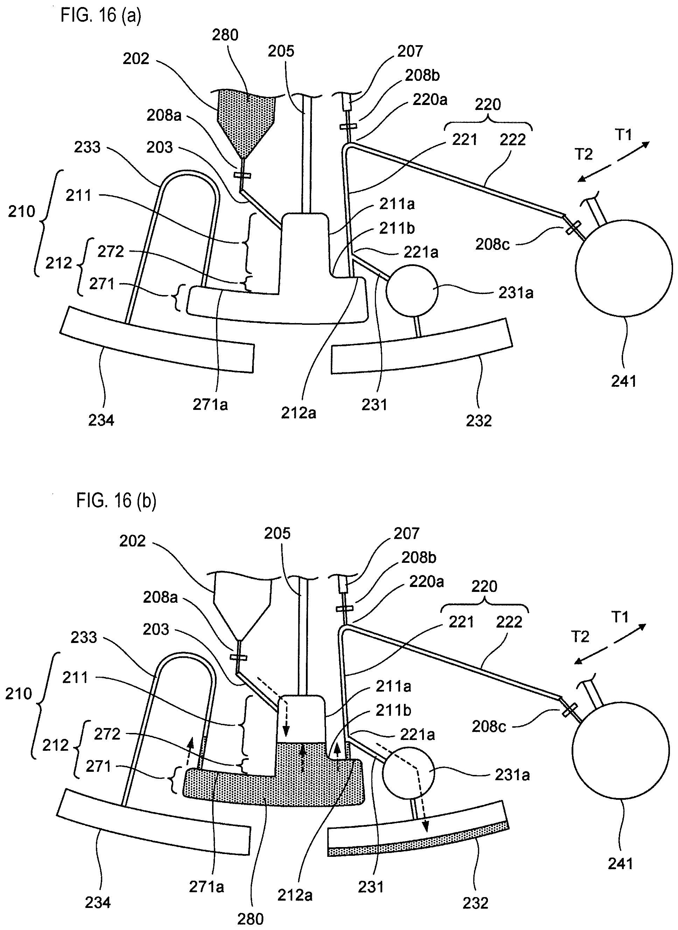

The process in step S11 will be described below in detail with reference to FIG. 15. The flowchart of FIG. 15 is a flowchart showing in detail the process of step S11 of FIG. 14. In the following description, referring primarily to FIG. 15, refer to the state transition diagrams of FIGS. 16 (a) to 18 (b) as appropriate.

Before the process of step S11 of FIG. 14 and step S101 of FIG. 15 starts, the sample 280 is accommodated in the sample storage part 202, as shown in FIG. 16 (a). In step S101, the controller 151 drives the motor 117 to rotate the measuring cartridge 200, and transfers the sample 280 in the sample storage part 202 to the separation chamber 210 by centrifugal force, as shown in FIG. 16 (b).

In the course of transferring the sample 280, the interface between the sample 280 and the air layer approaches the rotating shaft 103 in the separation chamber 210, the flow path 233, and the flow path 221. However, since the flow path 231 is connected to the flow path 221 at the branching position 221a, when the interface between the sample 280 and the air layer surpasses the branching position 221a inward in the radial direction, the sample 280 that surpasses the branching position 221a is discarded to the overflow chamber 232 through the flow path 231. In this way, even if the amount of the sample 280 introduced from the sample input port 201 varies, a predetermined amount of the sample 280 is stored in the separation chamber 210, and the interface between the sample 280 and the air layer in the separation chamber 210 is positioned at a predetermined position in the radial direction.

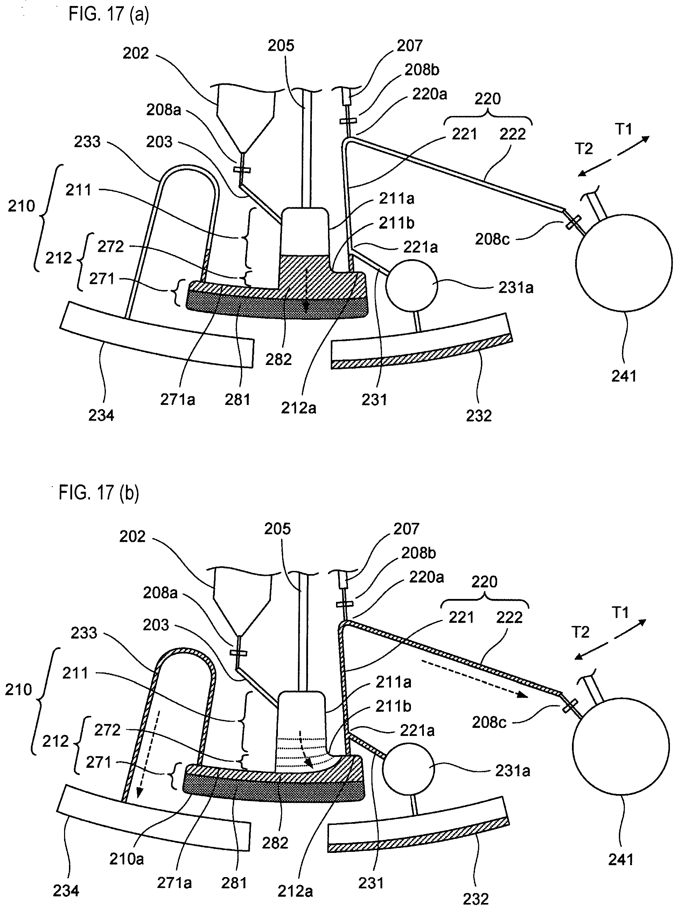

In step S102, the controller 151 drives the motor 117 to rotate the measuring cartridge 200, and the sample 280 in the separation chamber 210 undergoes separation by centrifugal force to separate the solid component 281 and the liquid component 282, as shown in FIG. 17 (a). At this time, the interface between the solid component 281 and the liquid component 282 is positioned in the first region 271. Here, since the flow path 220 extends radially inward from the separation chamber 210, the sample 280 that entered the flow path 221 prior to centrifuging smoothly moves from the flow path 221 to the separation chamber 210, as shown in FIG. 16 (b). Hence, it is possible to prevent the solid component 281 from remaining in the flow path 220 after centrifugal separation.

Subsequently, in step S103, the controller 151 waits for a predetermined time without rotating the measuring cartridge 200, whereby the liquid component 282 in the separation chamber 210 is transferred to the flow path 220 through capillary action, as shown in FIG. 17 (b). When the liquid component 282 in the separation chamber 210 moves to the flow path 220 in step S103, the interface between the air layer and the liquid component 282 gradually moves outward in the radial direction as indicated by a dotted line in FIG. 17 (b). At the same time, the liquid component 282 in the separation chamber 210 also fills the flow path 233 via capillary action.

Here, since the length of the first storage part 211 is shorter than the length of the second storage part 212 in the rotation direction, the interface between the air layer and the liquid component 282 appearing in the first storage part 211 after centrifugal separation is largely separated from the solid component 281. As described above, when the interface between the liquid component 282 and the air layer is largely separated from the liquid layer of the solid component 281, the position where the flow path 220 connects to the separation chamber 210 can be set to a position far away from the liquid layer of the solid component 281. In this way, it is possible to prevent the solid component 281 after centrifugation from mixing in the flow generated by capillary action in the flow path 50.

A curved inner wall 211b is provided between the inner wall 211 a of the first storage part 211 and the inner wall 212a of the second storage part 212. In this way, since the flow of the liquid component 282 smoothly changes along the curved inner wall 211b when the liquid component 282 moves from the first storage part 211 to the flow path 50 through capillary action, turbulence of the flow of liquid component 282 is prevented in the vicinity of the end part on the T2 direction side of the inner wall 212a. Therefore, it is possible to suppress mixing of the solid component 281 into the flow path 220 caused by such a turbulent flow.

The first storage part 211 is disposed at a position biased in the T2 direction relative to the second area 272, and the flow path 50 is connected at a position of the second area 272 on the T1 direction side relative to the first storage part 211, such that, while the liquid component 282 flows into the flow path 220 via capillary action, the interface between the air layer and the liquid component 282 travels in a direction away from the rotating shaft 103 and the end part of the interface on the flow path 220 side is inclined in a state closer to the rotating shaft 103 than the end part of the interface on the opposite side to the flow path 220 as shown by a dotted line in FIG. 17 (b). That is, the interface advances in a direction away from the rotating shaft 103 in a state in which the end part of the interface on the T1 direction side is closer to the inner side in the radial direction than the end part of the interface on the T2 direction side. In this way, it is possible to prevent the liquid component 282 from moving away from the connection position on the inner wall 212a of the flow path 220. Hence, the liquid component 282 can be stably supplied to the flow path 50 while the liquid component 282 flows into the flow path 50 via capillary action.

Note that, the interface between the air layer and the liquid component 282 proceeds in the T1 direction in the vicinity of the inner wall 212a. Therefore, in order to transfer a sufficient amount of the liquid component 282 to the flow path 220, it is preferable that the flow path 50 be connected to the T1 direction side of the inner wall 212a as far as possible.

The inner wall 271a also is longer than the inner wall 212a in the direction of rotation. In this case, the part of the first area 271 protruding in the T2 direction relative to the second area 272, that is, the protruding part 210a shown in FIG. 17 (b) is set long. The solid component 281 accumulated in the protruding part 210a by centrifugal separation is scarcely affected by the flow caused through capillary action and easily remains in the protruding part 210a after centrifugal separation. Therefore, the solid component 281 accumulated in the protruding part 210a by centrifugal separation scarcely enters the flow generated by capillary action. Accordingly, it is possible to increase the amount of the solid component 281 accumulated in the protruding part 210a by increasing the length of the inner wall 271a and increasing the length of the protruding part 210a. Hence, it is possible to more effectively prevent the solid component 281 after the centrifugation from flowing into the flow path 220 through capillary action.

A valve 208c is provided on the receiving chamber 241 side of the flow path 222. As shown in FIG. 17 (b), the valve 208c prevents the liquid component 282 that was transferred to the flow path 50 from moving from the flow path 50 to the receiving chamber 241. In this way, the liquid component 282 can accumulate in the range of the flow path 222 from the connection position 220a to the valve 208c through capillary action, and in the next step S 104, the liquid component 282 in a quantity defined in the range of the flow path 222 can be transferred to the receiving chamber 241. Hence, the quantitativeness of the liquid component 282 moving to the receiving chamber 241 is improved.

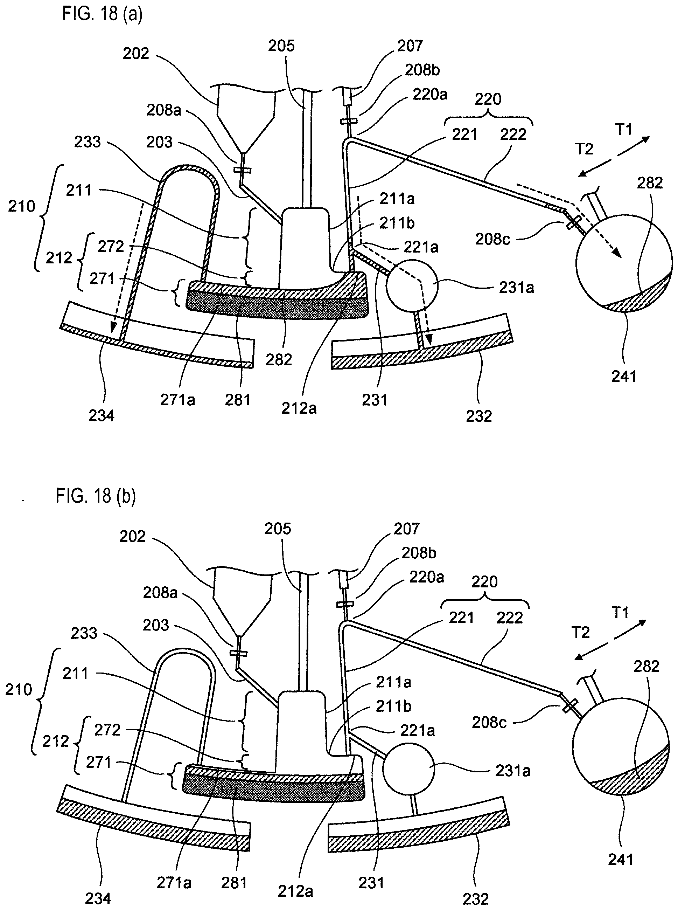

Subsequently, in step S104, the controller 151 drives the motor 117 to rotate the measuring cartridge 200, and transfers the liquid component 282 in the flow path 222 to the receiving chamber 241 by centrifugal force, as shown in FIGS. 18 (a) and 18 (b). FIG. 18 (a) is a diagram showing a state in the course of transferring the liquid component 282 in the flow path 222 to the receiving chamber 241, and FIG. 18 (b) is a diagram showing a state where the transfer of the liquid component 282 is completed. Thus, the liquid component 282 quantified by the flow path 222 is transferred to the receiving chamber 241.

Note that when the measuring cartridge 200 is rotated in step S104, the liquid component 282 in the flow path 221 is transferred to the overflow chamber 232 through the flow path 231 via centrifugal force. A part of the liquid component 282 in the flow path 221 also is returned to the separation chamber 210 by centrifugal force.

In step S104, when centrifugal force is applied to the measuring cartridge 200 from the state of FIG. 17 (b), the liquid component 282 in the separation chamber 210 Is transferred through the flow path 233 to the waste chamber 234 according to the siphon principle, as shown in FIG. 18 (a). In this way, as shown in FIG. 18 (b), the liquid component 282 remaining in the separation chamber 210 is transferred to the waste chamber 234, and the interface between the air layer and the liquid component 282 in the separation chamber 210 moves in a radially outward direction away from the connection position of the flow path 220 at the inner wall 212a. Accordingly, after moving the liquid component 282 from the separation chamber 210 to the receiving chamber 241, it is possible to prevent the liquid component 282 remaining in the separation chamber 210 from flowing back into the flow path 220 through capillary action toward the receiving chamber 241. Hence, the amount of the liquid component 282 transferred to the receiving chamber 241 can be stabilized, and the measurement accuracy of the liquid component 282 can be increased.

The flow path 233 also is connected to the inner wall 271a. In this way, when the liquid component 282 in the separation chamber 210 is discarded into the waste chamber 234, the solid component 281 can be prevented from mixing in the flow path 233 and blocking the flow path 233 with the solid component 281, as shown in FIG. 18 (a).

Returning to FIG. 14, the controller 151 transfers the reagent to the receiving chamber in step S12. Specifically, the controller 151 drives the motor 117 to rotate the measuring cartridge 200, and positions the sealing bodies 261a and 261b aligned in the radial direction just below the two pressing parts 124. Then, the controller 151 drives the two pressing parts 124 to push down the sealing bodies 261a and 261b to open the sealing bodies 261a and 261b. The controller 151 repeats the opening operation to unseal the six sealing bodies 261a and the six sealing bodies 261b positioned in the radial direction of the receiving chambers 241 to 246. Then, the controller 151 drives the motor 117 to rotate the measuring cartridge 200, and centrifugal force causes the reagents accommodated in the six liquid storage parts 261 located in the radial direction of the receiving chambers 241 to 246 to flow, respectively, to the receiving chambers 241 to 246 through the flow path 250.

In this way, the R1 reagent is transferred to the receiving chamber 241, and the liquid component, the R1 reagent, and the R2 reagent are mixed in the receiving chamber 241. The R3 reagent is transferred to the receiving chamber 242, the cleaning liquid is transferred to the receiving chambers 243 to 245, and the R4 reagent is transported to the receiving chamber 246.

When the transfer of the reagent is completed in step S12, the controller 151 then performs an agitation process. Specifically, the controller 151 drives the motor 117 so as to switch between two different rotation speeds at predetermined time intervals while rotating in a predetermined direction. In this way, the Euler force generated in the rotation direction changes at predetermined time intervals, whereby the liquid in the receiving chambers 241 to 246 is agitated. Such an agitation process is performed not only in step S12 but also in steps S13 to S18 in the same manner after the transfer process.

In this case, the R1 reagent contains a capture substance that binds to the target substance. The capture substance includes, for example, an antibody that binds to the target substance. The antibody is, for example, a biotin-conjugated HBs monoclonal antibody. The R2 reagent contains magnetic particles and magnetic particle suspension. Magnetic particles are, for example, streptavidin-bound magnetic particles whose surface is coated with avidin. When the liquid component separated from the sample, the R1 reagent, and the R2 reagent are mixed and agitated in step S12, the target substance and the R1 reagent are bound by an antigen-antibody reaction. Then, due to the reaction between the antigen-antibody reactants and the magnetic particles, the target substance bound to the capture substance of the R1 reagent binds to the magnetic particle via the capture substance. In this way, a complex is generated in a state where the target substance and the magnetic particles are bonded.

Next, in step S13, the controller 151 transfers the complex in the receiving chamber 241 from the receiving chamber 241 to the receiving chamber 242.

Specifically, the controller 151 drives the motor 117 to rotate the measuring cartridge 200, and positions the receiving chamber 241 just above the magnet of the magnetic force applicator 114. The controller 151 drives the magnetic force applicator 114 to bring the magnet closer to the lower surface of the measuring cartridge 200 to collect the complex spread in the receiving chamber 241. The controller 151 drives the magnetic force applicator 114 to move the magnet inward in the radial direction and transfer the complex in the receiving chamber 241 to the arcuate area of the flow path 250. The controller 151 drives the motor 117 to rotate the measuring cartridge 200 and transfers the complex along the arcuate area of the flow path 250. The controller 151 drives the magnetic force applicator 114 to move the magnet radially outward to transfer the complex to the receiving chamber 242. Then, the controller 151 drives the magnetic force applicator 114 to separate the magnet from the lower surface of the measuring cartridge 200.

The process of step S13 is performed in this way. Note that the transfer of the complex in steps S14 to S17 is also performed in the same manner as in step S13.

Thus, the complex generated in the receiving chamber 241 is mixed with the R3 reagent in the receiving chamber 242. In this case, the R3 reagent contains a labeling substance. The labeling substance includes a label and a capture substance that specifically binds to the target substance. For example, the labeling substance is a labeled antibody in which an antibody is used as a capture substance. In step S13, when the R3 reagent and the complex generated in the receiving chamber 241 are mixed and agitated, the complex reacts with the labeled antibody contained in the R3 reagent. In this way, a complex is generated in which the target substance, the capture antibody, the magnetic particles, and the labeled antibody are bound.

In step S14, the controller 151 transfers the complex in the receiving chamber 242 from the receiving chamber 242 to the receiving chamber 243. In this way, the cleaning liquid and the complex generated in the receiving chamber 242 are mixed in the receiving chamber 243. In step S14, when the cleaning liquid and the complex material generated in the receiving chamber 242 are mixed and agitated, the complex and the unreacted substance are separated in the receiving chamber 243. That is, unreacted substances are removed by cleaning in the receiving chamber 243.

In step S15, the controller 151 transfers the complex in the receiving chamber 243 from the receiving chamber 243 to the receiving chamber 244. In this way, the complex generated in the receiving chamber 242 is mixed with the cleaning liquid in the receiving chamber 244. Even in the receiving chamber 244, unreacted substances are removed by cleaning.

In step S16, the controller 151 transfers the complex in the receiving chamber 244 from the receiving chamber 244 to the receiving chamber 245. In this way, the complex generated in the containing chamber 242 is mixed with the cleaning liquid in the receiving chamber 245. Even in the receiving chamber 245, unreacted substances are removed by cleaning.

In step S17, the controller 151 transfers the complex in the receiving chamber 245 from the receiving chamber 245 to the receiving chamber 246. In this way, the complex generated in the receiving chamber 242 is mixed with the R4 reagent in the receiving chamber 246. In this case, the R4 reagent is a reagent for dispersing the complex generated in the receiving chamber 242. The R4 reagent is, for example, a buffer solution. In step S17, when the complex generated in the receiving chamber 242 and the R4 reagent are mixed and agitated, the complex generated in the receiving chamber 242 is dispersed.

In step S18, the controller 151 transfers the R5 reagent to the receiving chamber 246. Specifically, the controller 151 drives the motor 117 to rotate the measuring cartridge 200, and positions the sealing bodies 261a and 261b disposed closest to the T1 direction directly below the two pressing parts 124. Then, the controller 151 drives the two pressing parts 124 to press down the sealing bodies 261a and 261b, and the sealing bodies 261a and 261b are opened. Then, the controller 151 drives the motor 117 to rotate the measuring cartridge 200, and centrifugal force causes the R5 reagent accommodated in the liquid storage part 261 located closest to the T1 direction to flow through the flow path 250 to the receiving chamber 246. In this way, the R5 reagent is further mixed with the mixed solution generated in step S17 in the receiving chamber 246.

In this case, the R5 reagent is a luminescent reagent including a luminescent substrate that produces light upon reaction with a labeled antibody bound to the complex. In step S18, a sample is prepared when the mixed solution produced in step S17 and the R5 reagent are mixed and agitated. This sample chemiluminesces by reacting the labeling substance bound to the complex with the luminescent substrate.

In step S19, the controller 151 drives the motor 117 to rotate the measuring cartridge 200, positions the receiving chamber 246 right above the photodetector of the detecting unit 115, and detects the light generated from the receiving chamber 246 by photodetector. In step S20, the controller 151 performs analysis processing related to immunity based on the light detected by the photodetector of the detection unit 115. When the photodetector of the detection unit 115 is composed of a photomultiplier tube, a pulse waveform corresponding to photon reception is output from the photodetector. The detection unit 115 counts photons at regular intervals based on the output signal of the photodetector and outputs a count value. Based on the count value output from the detection unit 115, the controller 151 analyzes the presence/absence and quantity of the target substance, and displays the analysis result on the display unit 152.

### Fourth Embodiment

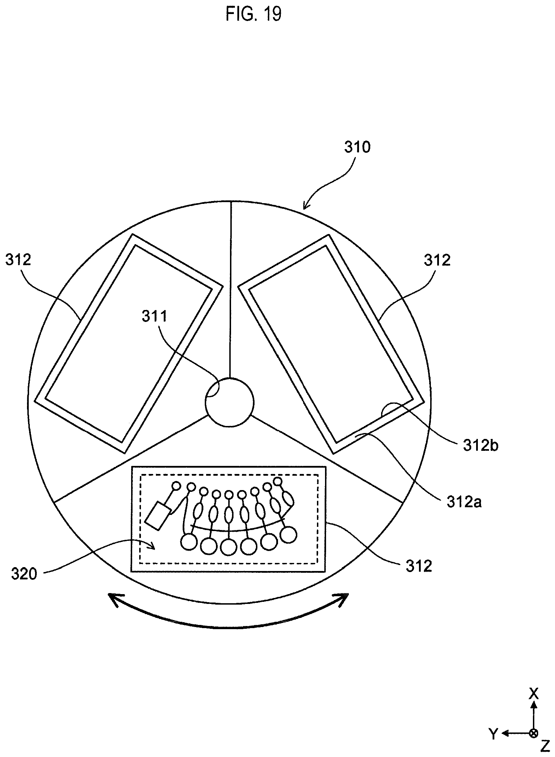

In the fourth embodiment shown in FIG. 19, a support member 310 is provided instead of the support member 113, and a measuring cartridge 320 is used instead of the measuring cartridge 200. Other aspects of the configuration are the same as the above specific configuration example.

The support member 310 includes a hole 311 and three mounting parts 312. The hole 311 is provided at the center of the support member 310. The support member 310 is installed on the rotating shaft 103. In this way, the support member 310 can rotate around the rotating shaft 103. Three mounting parts 312 are provided in the rotation direction. The mounting part 312 includes a surface 312a and a hole 312b. The surface 312a is one level lower than the upper surface of the support member 310. The hole 312b is formed at the center of the surface 312a and penetrates the support member 310 in the vertical direction. The measuring cartridge 320 has a rectangular shape. The measuring cartridge 320 has the same configuration as the measuring cartridge 200 except for the shape of the outer shape.

As in the case of the measuring cartridge 200, the operator inserts the sample into the sample input port of the measuring cartridge 320, and installs the measuring cartridge 320 in the mounting part 312 when starting the measurement. Then, similar to the above specific configuration example, the controller 151 drives the motor 117, the magnetic force applicator 114, and the detection unit 115. In the third embodiment, the measuring cartridges 320 can be installed on the three mounting parts 312, respectively, so that the three measuring cartridges 320 simultaneously measure.

### EXPLANATION OF THE REFERENCE NUMERALS

10, 200, 320 Measuring cartridge
20, 103 Rotating shaft
30, 210 Separation chamber
31, 211 First storage part
31a, 31b, 211a Inner wall
32, 212 Second storage part
32a, 32b, 212a Inner wall
40, 241 Receiving chamber
50, 220 Flow path
50a, 220a Connection position
51, 221 Flow path
52, 22 Flow path
61, 201 Sample input port
63, 203 Flow path
65, 207 Air Introduction Path
70,280 Sample
71, 281 Solid component
72, 282 Liquid component
81, 271 First area
81a, 81b, 271a Inner wall
82, 272 Second area
100 measuring device
208c valve
211b Inner wall
233 Flow path
234 Waste chamber
274a First inclined part
274b Second inclined part
274c Flat section

## Claims

1. A measuring cartridge (10, 200, 320) mountable on a measuring device (100) that is rotatable about a rotating shaft (20, 103), the measuring cartridge (10, 200, 320) comprising:
a sample input port (61, 201) for inputting a blood sample;
a separation chamber (30, 210) for separating the blood sample into a blood cell component and a plasma component by utilizing centrifugal force through rotation about the rotating shaft (20, 103), comprising a first storage part (31, 211), and a second storage part (32, 212) arranged in a direction away from the rotating shaft (20, 103) relative to the first storage part (31, 211) and having a width in an rotation direction around the rotating shaft (20, 103) larger than that of the first storage part (31, 211); the first storage part (31, 211) is disposed at a position biased relative to the second storage part (32, 212) in the rotation direction,
a receiving chamber (40, 241) for containing the plasma component; and
a flow path (50, 63) connected to an inner wall of at least one of the first storage part and the second storage part;
wherein the flow path (50, 63) comprises a first flow path (63) for moving the blood sample introduced through the sample input port to the separation chamber (30, 210), and a second flow path (50) for moving the plasma component separated in the separation chamber (30, 210) by a capillary phenomenon.

2. The measuring cartridge according to claim 1, wherein
the second flow path (50) is connected to a first inner wall (32a) located at an end on the rotating shaft side of the second storage part (32, 212).

3. The measuring cartridge according to claim 2, wherein
the second inner wall of the first storage part (31, 211) positioned on the opposite side of the rotating shaft from the end portion of the first storage part (31, 211) positioned on the rotating shaft side is connected to the first inner wall by a curved inner wall inclined so as to become gradually parallel to the first inner wall from the end edge of the second inner wall and connected to the first inner wall (32a).

4. The measuring cartridge according to claim 1, wherein
the second flow path (50) is connected to the inner wall of the first storage part (31, 211) positioned on the side opposite to the rotating shaft with respect to the end section of the first storage part (31, 211) positioned on the rotating shaft side.

5. The measuring cartridge of any one of claims 1 to 4, wherein
the second flow path (50) extends in a direction from the separation chamber toward the rotating shaft.

6. The measuring cartridge of any one of claims 1 to 5, wherein
the second storage part (32, 212) comprises a first area (81), and a second area (82) disposed on the rotating shaft side relative to the first area (81) and in which the width in the rotation direction is smaller than the first area (81) .

7. The measuring cartridge of any one of claims 1 to 6, wherein
the second flow path (50) comprises a third flow path (51, 221) connecting the separation chamber (30, 120) and the receiving chamber (40, 241) and extending in a direction from the separation chamber to the rotating shaft, and a fourth flow path (52, 222) extending in a direction away from the rotating shaft from the end on the opposite side to the separation chamber in the third flow path (51, 221).

8. The measuring cartridge according to claim 7, wherein
an air introduction path (65, 207) capable of introducing air into the second flow path is connected to the connection position between the third flow path (51, 221) and the fourth flow path (52, 222).

9. The measuring cartridge according to claim 7 or 8, wherein
a valve (208c) is provided on the receiving chamber side of the fourth flow path (52, 222) in order to stop the movement of the plasma component due to capillary action.

10. The measuring cartridge of any one of claims 1 to 9, wherein
the second storage part (32, 212) comprises a first inclined part (274a) that increases the thickness of the space in the second storage part as the distance from the rotating shaft increases.

11. The measuring cartridge of any one of claims 1 to 10, wherein
the second storage part (32, 212) comprises a second inclined part (274b) that reduces the thickness of the space in the second storage part as the distance from the rotating shaft increases.

12. The measuring cartridge of any one of claims 1 to 11, wherein
the second storage part (32, 212) comprises a first inclined part (274a) that increases the thickness of the space in the second storage part as the distance increases from the rotating shaft, and a second inclined part (274b) provided on the side closer to the rotating shaft relative to the first inclined part, that reduces the thickness of the space in the second storage part (32, 212) as the distance increases from the rotating shaft.

13. A liquid transport method using a measuring cartridge mountable on a measuring device that is rotatable about a rotating shaft, the method comprising:
moving a blood sample to a separation chamber (30, 210) comprising a first storage part (31, 211) and a second storage part (32, 212) having a larger width in the rotation direction around the rotating shaft than that of the first storage part (31, 211) using a first flow path (63) connected to the inner wall of the first storage part or the second storage part;
separating the blood sample in the separation chamber (30, 210) into a blood cell component and a plasma component using centrifugal force or rotation about a rotating shaft; and
moving the separated plasma component by capillary action using a second flow path (50) connected to the inner wall of the first storage part (31, 211) or the second storage part (32, 212),
the measuring cartridge being the measuring cartridge of one of claims 1 to 12.

## Patentansprüche

1. Messpatrone (10, 200, 320), die an einer Messvorrichtung (100) montierbar ist, die um eine Drehwelle (20, 103) drehbar ist, wobei die Messpatrone (10, 200, 320) Folgendes umfasst:
eine Probeneingabeöffnung (61, 201) zum Eingeben einer Blutprobe;
eine Trennkammer (30, 210) zum Trennen der Blutprobe in eine Blutzellenkomponente und eine Plasmakomponente durch Einsetzen von Zentrifugalkraft durch Drehen um die Drehwelle (20, 103), die ein erstes Lagerteil (31, 211) und ein zweites Lagerteil (32, 212) umfasst, das in einer Richtung von der Drehwelle (20, 103) in Bezug auf das erste Lagerteil (31, 211) weg eingerichtet ist und eine Breite in einer Drehrichtung um die Drehwelle (20, 103) aufweist, die größer ist als die des ersten Lagerteils (31, 211); wobei das erste Lagerteil (31, 211) an einer Position angeordnet ist, die in Bezug auf das zweite Lagerteil (32, 212) in der Drehrichtung vorgespannt ist,
eine Aufnahmekammer (40, 241) zum Enthalten der Plasmakomponente; und
einen Flussweg (50, 63), der mit einer Innenwand mindestens eines des ersten Lagerteils und des zweiten Lagerteils verbunden ist;
wobei der Flussweg (50, 63) einen ersten Flussweg (63) zum Bewegen der Blutprobe, die durch die Probeeingabeöffnung zu der Trennkammer (30, 210) eingeführt wurde, umfasst, und einen zweiten Flussweg (50) zum Bewegen der Plasmakomponente, die in der Trennkammer (30, 210) durch eine Kapillarerscheinung getrennt wird.

2. Messpatrone nach Anspruch 1, wobei
der zweite Flussweg (50) mit einer ersten Innenwand (32a), die an einem Ende auf der Drehwellenseite des zweiten Lagerteils (32, 212) liegt, verbunden ist.

3. Messpatrone nach Anspruch 2, wobei
die zweite Innenwand des ersten Lagerteils (31, 211), die auf der entgegengesetzten Seite der Drehwelle von dem Endabschnitt des ersten Lagerteils (31, 211), das auf der Drehwellenseite positioniert ist, positioniert ist, mit der ersten Innenwand durch eine gekrümmte Innenwand verbunden ist, die derart geneigt ist, dass sie allmählich zu der ersten Innenwand von der Endkante der zweiten Innenwand parallel wird, und mit der ersten Innenwand (32a) verbunden ist.

4. Messpatrone nach Anspruch 1, wobei
der zweite Flussweg (50) mit der Innenwand des ersten Lagerteils (31, 211) an der Seite entgegengesetzt zu der Drehwelle in Bezug auf den Endabschnitt des ersten Lagerteils (31, 211), der auf der Drehwellenseite positioniert ist, positioniert ist.

5. Messpatrone nach einem der Ansprüche 1 bis 4, wobei
sich der zweite Flussweg (50) in einer Richtung von der Trennkammer zu der Drehwelle erstreckt.

6. Messpatrone nach einem der Ansprüche 1 bis 5, wobei
das zweite Lagerteil (32, 212) eine erste Fläche (81) umfasst, und eine zweite Fläche (82), die auf der Drehwellenseite in Bezug auf die erste Fläche (81) angeordnet ist, und in der die Breite in der Drehrichtung kleiner ist als die erste Fläche (81).

7. Messpatrone nach einem der Ansprüche 1 bis 6, wobei
der zweite Flussweg (50) einen dritten Flussweg (51, 221) umfasst, der die Trennkammer (30, 120) und die Aufnahmekammer (40, 241) verbindet und sich in einer Richtung von der Trennkammer zu der Drehwelle erstreckt, und einen vierten Flussweg (52, 222), der sich in einer Richtung von der Drehwelle von dem Ende auf der entgegengesetzten Seite zu der Trennkammer in dem dritten Flussweg (51, 221) erstreckt.

8. Messpatrone nach Anspruch 7, wobei
ein Lufteinführungsweg (65, 207), der fähig ist, Luft in den zweiten Flussweg einzuführen, mit der Verbindungsposition zwischen dem dritten Flussweg (51, 221) und dem vierten Flussweg (52, 222) verbunden ist.

9. Messpatrone nach Anspruch 7 oder 8, wobei
ein Ventil (208c) an der Aufnahmekammerseite des vierten Flusswegs (52, 222) bereitgestellt ist, um die Bewegung der Plasmakomponente aufgrund von Kapillarwirkung zu stoppen.

10. Messpatrone nach einem der Ansprüche 1 bis 9, wobei
das zweite Lagerteil (32, 212) ein erstes geneigtes Teil (274a) umfasst, das das Ausmaß des Raums in dem zweiten Lagerteil mit zunehmender Entfernung von der Drehwelle steigert.

11. Messpatrone nach einem der Ansprüche 1 bis 10, wobei
das zweite Lagerteil (32, 212) ein zweites geneigtes Teil (274b) umfasst, das das Ausmaß des Raums in dem zweiten Lagerteil mit zunehmender Entfernung von der Drehwelle reduziert.

12. Messpatrone nach einem der Ansprüche 1 bis 11, wobei
das zweite Lagerteil (32, 212) ein erstes geneigtes Teil (274a) umfasst, das das Ausmaß des Raums in dem zweiten Lagerteil mit zunehmender Entfernung von der Drehwelle steigert, und ein zweites geneigtes Teil (274b), das an der Seite bereitgestellt ist, die der Drehwelle in Bezug auf das erste geneigte Teil näher liegt, das das Ausmaß des Raums in dem zweiten Lagerteil (32, 212) mit zunehmender Entfernung von der Drehwelle reduziert.

13. Flüssigkeitstransportverfahren, das eine Messpatrone umfasst, die an einer Messvorrichtung montierbar ist, die um eine Drehwelle drehbar ist, wobei das Verfahren Folgendes umfasst:
Bewegen einer Blutprobe zu einer Trennkammer (30, 210), die ein erstes Lagerteil (31, 211) und ein zweites Lagerteil (32, 212) umfasst, das eine größere Breite in der Drehrichtung um die Drehwelle aufweist als die des ersten Lagerteils (31, 211), indem ein erster Flussweg (63), der mit der Innenwand des ersten Lagerteils oder des zweiten Lagerteils verbunden ist, verwendet wird;
Trennen der Blutprobe in der Trennkammer (30, 210) in eine Blutzellenkomponente und eine Plasmakomponente unter Verwenden von Zentrifugalkraft oder Drehung um eine Drehwelle; und
Bewegen der getrennten Plasmakomponente durch Kapillarwirkung unter Verwenden eines zweiten Flusswegs (50), der mit der Innenwand des ersten Lagerteils (31, 211) oder des zweiten Lagerteils (32, 212) verbunden ist,
wobei die Messpatrone die Messpatrone eines der Ansprüche 1 bis 12 ist.

## Revendications

1. Cartouche de mesure (10, 200, 320) pouvant être montée sur un dispositif de mesure (100) qui peut tourner autour d'un arbre rotatif (20, 103), la cartouche de mesure (10, 200, 320) comprenant :
un orifice d'entrée d'échantillon (61, 201) pour entrer un échantillon sanguin ;
une chambre de séparation (30, 210) pour séparer l'échantillon sanguin en un composant globule sanguin et en un composant plasma en utilisant une force centrifuge par rotation autour de l'arbre rotatif (20, 103), comprenant une première partie de stockage (31, 211), et une seconde partie de stockage (32, 212) agencée dans une direction s'éloignant de l'arbre rotatif (20, 103) par rapport à la première partie de stockage (31, 211) et présentant une largeur dans une direction de rotation autour de l'arbre rotatif (20, 103) supérieure à celle de la première partie de stockage (31, 211) ; la première partie de stockage (31, 211) est disposée dans une position sollicitée par rapport à la seconde partie de stockage (32, 212) dans la direction de rotation,
une chambre de réception (40, 241) pour contenir le composant plasma ; et
un chemin d'écoulement (50, 63) relié à une paroi intérieure d'au moins une de la première partie de stockage et de la seconde partie de stockage ;
dans laquelle le chemin d'écoulement (50, 63) comprend un premier chemin d'écoulement (63) pour déplacer l'échantillon sanguin introduit par l'orifice d'entrée d'échantillon dans la chambre de séparation (30, 210), et un deuxième chemin d'écoulement (50) pour déplacer le composant plasma séparé dans la chambre de séparation (30, 210) par un phénomène capillaire.

2. Cartouche de mesure selon la revendication 1, dans laquelle
le deuxième chemin d'écoulement (50) est relié à une première paroi intérieure (32a) située au niveau d'une extrémité sur le côté arbre rotatif de la seconde partie de stockage (32, 212).

3. Cartouche de mesure selon la revendication 2, dans laquelle
la seconde paroi intérieure de la première partie de stockage (31, 211) positionnée sur le côté opposé de l'arbre rotatif par rapport à la partie d'extrémité de la première partie de stockage (31, 211) positionnée sur le côté arbre rotatif est reliée à la première paroi intérieure par une paroi intérieure incurvée inclinée de manière à devenir progressivement parallèle à la première paroi intérieure depuis le bord d'extrémité de la seconde paroi intérieure et reliée à la première paroi intérieure (32a).

4. Cartouche de mesure selon la revendication 1, dans laquelle
le deuxième chemin d'écoulement (50) est relié à la paroi intérieure de la première partie de stockage (31, 211) positionnée sur le côté opposé à l'arbre rotatif par rapport à la section d'extrémité de la première partie de stockage (31, 211) positionnée sur le côté arbre rotatif.

5. Cartouche de mesure selon l'une quelconque des revendications 1 à 4, dans laquelle
le deuxième chemin d'écoulement (50) s'étend dans une direction depuis la chambre de séparation vers l'arbre rotatif.

6. Cartouche de mesure selon l'une quelconque des revendications 1 à 5, dans laquelle
la seconde partie de stockage (32, 212) comprend une première zone (81), et une seconde zone (82) disposée sur le côté arbre rotatif par rapport à la première zone (81) et dans laquelle la largeur dans la direction de rotation est inférieure à la première zone (81).

7. Cartouche de mesure selon l'une quelconque des revendications 1 à 6, dans laquelle
le deuxième chemin d'écoulement (50) comprend un troisième chemin d'écoulement (51, 221) reliant la chambre de séparation (30, 120) et la chambre de réception (40, 241) et s'étendant dans une direction allant de la chambre de séparation à l'arbre rotatif, et un quatrième chemin d'écoulement (52, 222) s'étendant dans une direction s'éloignant de l'arbre rotatif depuis l'extrémité sur le côté opposé vers la chambre de séparation dans le troisième chemin d'écoulement (51, 221).

8. Cartouche de mesure selon la revendication 7, dans laquelle
un chemin d'introduction d'air (65, 207) capable d'introduire de l'air dans le deuxième chemin d'écoulement est relié à la position de liaison entre le troisième chemin d'écoulement (51, 221) et le quatrième chemin d'écoulement (52, 222).

9. Cartouche de mesure selon la revendication 7 ou 8, dans laquelle
un clapet (208c) est prévu sur le côté chambre de réception du quatrième chemin d'écoulement (52, 222) afin d'arrêter le déplacement du composant plasma dû à une action capillaire.

10. Cartouche de mesure selon l'une quelconque des revendications 1 à 9, dans laquelle
la seconde partie de stockage (32, 212) comprend une première partie inclinée (274a) qui augmente l'épaisseur de l'espace dans la seconde partie de stockage à mesure que la distance depuis l'arbre rotatif augmente.

11. Cartouche de mesure selon l'une quelconque des revendications 1 à 10, dans laquelle
la seconde partie de stockage (32, 212) comprend une seconde partie inclinée (274b) qui réduit l'épaisseur de l'espace dans la seconde partie de stockage à mesure que la distance depuis l'arbre rotatif augmente.

12. Cartouche de mesure selon l'une quelconque des revendications 1 à 11, dans laquelle
la seconde partie de stockage (32, 212) comprend une première partie inclinée (274a) qui augmente l'épaisseur de l'espace dans la seconde partie de stockage à mesure que la distance depuis l'arbre rotatif augmente, et une seconde partie inclinée (274b) prévue sur le côté plus proche de l'arbre rotatif par rapport à la première partie inclinée, qui réduit l'épaisseur de l'espace dans la seconde partie de stockage (32, 212) à mesure que la distance depuis l'arbre rotatif augmente.

13. Procédé de transport de liquide utilisant une cartouche de mesure pouvant être montée sur un dispositif de mesure qui peut tourner autour d'un arbre rotatif, le procédé comprenant :
le déplacement d'un échantillon sanguin vers une chambre de séparation (30, 210) comprenant une première partie de stockage (31, 211) et une seconde partie de stockage (32, 212) présentant une largeur dans la direction de rotation autour de l'arbre rotatif supérieure à celle de la première partie de stockage (31, 211) en utilisant un premier chemin d'écoulement (63) relié à la paroi intérieure de la première partie de stockage ou la seconde partie de stockage ;
la séparation de l'échantillon sanguin dans la chambre de séparation (30, 210) en un composant globule sanguin et en un composant plasma à l'aide d'une force centrifuge ou d'une rotation autour d'un arbre rotatif ; et
le déplacement du composant plasma séparé par action capillaire à l'aide d'un deuxième chemin d'écoulement (50) relié à la paroi intérieure de la première partie de stockage (31, 211) ou la seconde partie de stockage (32, 212),
la cartouche de mesure étant la cartouche de mesure selon l'une des revendications 1 à 12.
